# EUROPÄISCHE PATENTSCHRIFT

(11) **EP 2 510 887 B1**
(45) Veröffentlichungstag und Bekanntmachung des Hinweises auf die Patenterteilung: **23.03.2016**
(21) Anmeldenummer: 12002090.4
(22) Anmeldetag: 24.03.2012
(51) Int. Cl.: A61B 17/29

(54) **Werkzeug für ein mikroinvasives-chirurgisches Instrument**
Tool for a micro-surgical instrument
Outil pour un instrument de chirurgie micro-invasive

(30) Priorität: 11.04.2011 DE 102011007122
(43) Veröffentlichungstag der Anmeldung: 17.10.2012
(73) Patentinhaber: Karl Storz GmbH & Co. KG, 78532 Tuttlingen (DE)
(72) Erfinder: Stefan, Jochen, 78532 Tuttlingen (DE); Kärcher, Daniel, 78532 Tuttlingen (DE); Bacher, Uwe, 78532 Tuttlingen (DE)

(56) Entgegenhaltungen:
- EP-A2- 1 889 579
- DE-C1- 19 707 373
- DE-U1- 9 317 535
- US-A- 5 893 875

## Beschreibung

Die vorliegende Erfindung bezieht sich auf ein Werkzeug für ein mikroinvasiv-chirurgisches Instrument, ein mikroinvasiv-chirurgisches Instrument und dabei insbesondere auf Merkmale zur mechanischen Kopplung des Werkzeugs mit einem distalen Ende eines Schafts.

Viele mikroinvasiv-chirurgische Instrumente umfassen einen langen und dünnen Schaft, ein Werkzeug am distalen Ende des Schafts und eine Handhabungseinrichtung am proximalen Ende des Schafts. Das Werkzeug umfasst beispielsweise eine Fass-, Präparier-, Biopsie- oder andere Zange, eine Schere oder einen Nadelhalter mit mindestens zwei geraden oder gekrümmten Backen, Schneiden oder anderen Maulteilen, von denen mindestens eine bzw. eines bewegbar ist. Alternativ umfasst das Werkzeug eine andere Wirkeinrichtung, beispielsweise einen Manipulator mit einem Finger bzw. einer fingerförmigen Einrichtung oder eine Elektrode in Hakenform oder in anderer Gestalt. Der Schaft enthält (mindestens) eine Übertragungsstange, die in der Regel in einem geschlossenen Kanal im Inneren des Schafts angeordnet ist. Die Handhabungseinrichtung umfasst ein oder mehrere relativ zueinander bewegbare Betätigungseinrichtungen, beispielsweise zwei Griffteile, die medizinisches Personal mit einer Hand relativ zueinander bewegen kann. Das proximale Ende und das distale Ende der Übertragungsstange sind so mit der Betätigungseinrichtung bzw. mit dem Werkzeug gekoppelt, dass eine vom medizinischen Personal auf die Betätigungseinrichtungen ausgeübte Kraft oder eine durch medizinisches Personal hervorgerufene relative Bewegung der Betätigungseinrichtungen auf das Werkzeug übertragen werden, beispielsweise um Backen aufeinander zu zu bewegen bzw. zusammenzudrücken.

Bei der Verwendung eines derartigen mikroinvasiv-chirurgischen Instruments werden das Werkzeug und ein Teil des Schafts beispielsweise durch eine natürliche oder künstliche Körperöffnung in einen natürlichen oder künstlichen Hohlraum im Körper eines Patienten eingeführt. Die Entwicklung mikroinvasiver Operationstechniken geht dahin, immer kleinere und vor allem immer weniger Zugänge zu verwenden. Um beispielsweise in der laparoskopischen Chirurgie durch einen einzigen Trokar hindurch mit einem Endoskop und zwei Instrumenten arbeiten zu können, können Instrumente mit gekrümmten Schäften verwendet werden. Ein Instrument mit einem gekrümmten Schaft kann allerdings innerhalb des Zugangs nicht ohne weiteres um seine Längsachse gedreht werden, um die Orientierung des Werkzeugs an seinem distalen Ende zu verändern.

In der DE 10 2006 038 516 A1 ist ein medizinisches Rohrschaftinstrument beschrieben, bei dem ein Werkzeug 5, ein Schaft 3 und eine Handhabe 2 zur Reinigung voneinander getrennt werden. Dieses Instrument wird als nächstliegender Stand der Technik betrachtet, und offenbart eine Verriegelungseinrichtung mit einer Arbeitsposition und axial dazu verschobener Montageposition, wobei die Verriegelungseinrichtung auf einer Bajonettverbindung mit einer selbstsichernden Zapfen-Schlitz-Steuerung basiert.

In der DE 10 2008 015 418 A1 ist ein medizinisches Instrument mit einem gekrümmten Schaft beschrieben. Ein Maulteil ist mittels eines Bajonettverschlusses mit einem Schaft lösbar verbunden. Im verbundenen Zustand ist das Maulteil gegenüber dem Schaft drehbar. Der Schaft ist lösbar mit einer Handhabe verbunden. Mittels eines Handrads, das drehfest mit einem äußeren Schaftrohr verbunden ist, kann der gekrümmte Schaft gegenüber der Handhabe gedreht werden. Ein Innenrohr ist mit einem weiteren Handrad an der Handhabe verbunden. Das Instrument kann als unipolares oder bipolares HF-Instrument ausgebildet sein.

In der DE 10 2008 052 623 A1 ist ein chirurgisches Instrument mit einer Mauleinheit, einem Schaft und einer Griffeinheit beschrieben. Die Mauleinheit ist lösbar am Ende eines Schaftrohres des Schafts befestigt und gegenüber diesem drehbar.

Werkzeug, Schaft und Handhabungseinrichtung eines mikroinvasiv-chirurgischen Instruments sollen ohne Verwendung von Hilfsmitteln voneinander trennbar und miteinander verbindbar bzw. koppelbar sein, um eine einfache und gründliche Reinigung des Instruments zu ermöglichen. Beispielsweise aus der DE 10 2006 038 516 A1 ist es bekannt, das Werkzeug und das distale Ende des Schafts so auszubilden, dass das Werkzeug in einer über-offenen Montagestellung am Schaft montiert und demontiert werden kann. Insbesondere bei einer Drehbarkeit bzw. Rotierbarkeit des Werkzeugs gegenüber dem Schaft im gekoppelten Zustand sind jedoch einige Aspekte sowohl der Kopplung des Werkzeugs mit dem Schaft als auch der Kopplung des Schafts mit der Handhabungseinrichtung bislang nicht vollständig befriedigend gelöst.

Eine Aufgabe der vorliegenden Erfindung besteht darin, ein verbessertes Werkzeug für ein mikroinvasiv-chirurgisches Instrument und ein verbessertes mikroinvasiv-chirurgisches Instrument zu schaffen.

Diese Aufgabe wird durch den Gegenstand des unabhängigen Anspruchs gelöst.

Weiterbildungen sind in den abhängigen Ansprüchen angegeben.

Ein Werkzeug, das mit einem distalen Ende eines Schafts für ein mikroinvasiv-chirurgisches Instrument lösbar mechanisch koppelbar ist, umfasst eine Gelenkeinrichtung, an der ein Maulteil oder eine andere Wirkeinrichtung befestigt ist, ein Verbindungsbauteil, das mit der Gelenkeinrichtung rotierbar mechanisch verbunden ist, und das eine Kupplungseinrichtung zur lösbaren mechanischen Kupplung mit einem distalen Ende eines Schafts aufweist, eine Übertragungsstange zum Übertragen zumindest entweder einer Kraft oder eines Drehmoments von einem proximalen Ende eines mit dem Werkzeug lösbar mechanisch gekoppelten Schafts zu dem Maulteil oder der anderen Wirkeinrichtung und eine Verriegelungseinrichtung, die mit der Übertragungsstange derart mechanisch gekoppelt ist, dass die Verriegelungseinrichtung relativ zu der Übertragungsstange rotierbar, aber nicht axial verschiebbar ist, wobei die Verriegelungseinrichtung in dem Verbindungsbauteil derart mechanisch gelagert ist, dass die Verriegelungseinrichtung relativ zu dem Verbindungsbauteil axial verschiebbar, aber nicht rotierbar ist.

Das Werkzeug umfasst insbesondere eine Fass-, Präparier-, Biopsie- oder andere Zange, eine Schere oder einen Nadelhalter mit mindestens zwei geraden oder gekrümmten Backen, Schneiden oder anderen Maulteilen, von denen mindestens eine bzw. eines bewegbar ist. Das Maulteil kann entsprechend als Backe oder Schneide ausgebildet sein. Das Werkzeug kann zwei oder mehr Maulteile umfassen, die symmetrisch und synchron bewegt werden können. Alternativ umfasst das Werkzeug beispielsweise ein starr mit der Gelenkeinrichtung verbundenes und ein gelenkig an der Gelenkeinrichtung befestigtes Maulteil. Die Gelenkeinrichtung kann hinsichtlich der gelenkigen oder starren Befestigung von Maulteilen an der Gelenkeinrichtung ähnlich ausgebildet sein, wie es von Gabeleinrichtungen an herkömmlichen Werkzeugen für mikroinvasiv-chirurgischen Instrumenten bekannt ist. Alternativ umfasst das Werkzeug eine andere Wirkeinrichtung, beispielsweise einen Manipulator mit einem Finger bzw. einer fingerförmigen Einrichtung oder eine Elektrode in Hakenform oder in anderer Gestalt.

Das Verbindungsbauteil ist insbesondere hülsenförmig oder im Wesentlichen hülsenförmig und kann aus einem oder mehreren Elementen aufgebaut sein. Mit der Längsachse des Verbindungsbauteils, eines Schafts, einer Übertragungsstange oder eines proximalen Endes eines Schafts oder einer Übertragungsstange ist insbesondere die Achse gemeint, zu der der betreffende Gegenstand rotationssymmetrisch oder im Wesentlichen rotationssymmetrisch ist. Im Fall eines gekrümmten Schafts oder einer biegeflexiblen Übertragungsstange beziehen sich diese Aussagen insbesondere auf deren Enden, die in der Regel zumindest abschnittsweise gerade bzw. nicht gekrümmt sind.

Das Verbindungsbauteil kann eine Gestalt aufweisen, die von einer Rotationssymmetrie bezüglich einer Achse bzw. von einer Achsensymmetrie deutlich abweicht. Deutliche Abweichungen können insbesondere an der Kupplungseinrichtung vorliegen. Das Verbindungsbauteil weist jedoch insbesondere trotzdem Merkmale auf, die rotationssymmetrisch oder im Wesentlichen rotationssymmetrisch ausgebildet sind. Insbesondere weist das Verbindungsbauteil an seinem distalen Ende oder nahe seinem distalen Ende zu seiner Längsachse rotationssymmetrische Merkmale auf, über die es mit der Gelenkeinrichtung rotierbar mechanisch verbunden ist.

Das Verbindungsbauteil ist also einerseits mittels seiner Kupplungseinrichtung lösbar mechanisch mit dem distalen Ende eines Schafts koppelbar und andererseits um seine Längsachse rotierbar mit der Gelenkeinrichtung verbunden. Dadurch ermöglicht das Verbindungsbauteil eine Montierbarkeit am und eine Demontierbarkeit von einem distalen Ende eines Schafts und gleichzeitig eine Rotierbarkeit der Gelenkeinrichtung und vor allem der Maulteile oder anderen Wirkeinrichtungen um eine Längsachse des Werkzeugs und/oder um eine Längsachse des distalen Endes eines Schafts.

Insbesondere wenn das Werkzeug zur lösbaren mechanischen Kopplung mit einem gekrümmten Schaft vorgesehen ist, ist die Übertragungsstange biegeflexibel, um eine Rotation der Übertragungsstange im Schaft um ihre Längsachse und eine axiale Verschiebung der Übertragungsstange auch bei variierender Krümmung des Schafts zu ermöglichen. Die Verriegelungseinrichtung ist insbesondere um die lokale Längsachse der Übertragungsstange rotierbar. Das Merkmal, dass die Verriegelungseinrichtung gegenüber der Übertragungsstange nicht axial verschiebbar ist, umfasst auch den Fall, dass die Verriegelungseinrichtung gegenüber der Übertragungsstange im Wesentlichen nicht axial verschiebbar ist. Eine geringfügige axiale Verschiebbarkeit aufgrund eines mechanischen Spiels ist nicht ausgeschlossen.

Durch die Rotierbarkeit der Verriegelungseinrichtung gegenüber der Übertragungsstange und durch die Führung der Verriegelungseinrichtung in der Nut bzw. in dem Schlitz kann die Verriegelungsfunktion der Verriegelungseinrichtung von der Orientierung der Übertragungsstange hinsichtlich einer Rotation um ihre Längsachse unabhängig sein. Stattdessen ist die Wirkung der Verriegelungseinrichtung ausschließlich von der Position der Übertragungsstange hinsichtlich einer Verschiebung in Längsrichtung abhängig.

Die Übertragungsstange weist insbesondere ein elektrisch leitfähiges Material auf und ist elektrisch leitfähig mit einem Maulteil oder einer anderen Wirkeinrichtung des Werkzeugs verbunden. In diesem Fall können über die Übertragungsstange eine elektrische Spannung, ein Strom und elektrische Leistung zu dem Maulteil oder der anderen Wirkeinrichtung übertragen werden. Damit kann das Werkzeug beispielsweise zur HF-Koagulation verwendet werden.

Die Kupplungseinrichtung ist erfindungsgemäß als Bajonettkupplung ausgebildet.

Bei einem Werkzeug, wie es hier beschrieben ist, ist die Verriegelungseinrichtung in axialer Richtung zwischen einer Montageposition und einer Arbeitsposition verschiebbar, wobei in der Montageposition der Verriegelungseinrichtung das Verbindungsbauteil mit einem distalen Ende eines Schafts verbindbar und von diesem lösbar ist, und wobei in der Arbeitsposition der Verriegelungseinrichtung eine mechanische Verbindung des Werkzeugs mit einem distalen Ende eines Schafts verriegelt ist.

Die Verriegelungseinrichtung weist insbesondere mehrere Arbeitspositionen bzw. einen Bereich von Arbeitspositionen, der im Folgenden auch als Arbeitsbereich bezeichnet wird, auf. Die Montageposition der Verriegelungseinrichtung liegt insbesondere distal beabstandet von der Arbeitsposition bzw. von dem Arbeitsbereich. Die Verriegelungseinrichtung ist insbesondere linear parallel zur Längsachse des Werkzeugs verschiebbar. Zusätzlich ist die Verriegelungseinrichtung rotierbar. Insbesondere ist die Verriegelungseinrichtung zusammen mit dem Verbindungsbauteil relativ zur Gelenkeinrichtung des Werkzeugs rotierbar.

Mit einer Verriegelungseinrichtung, die in axialer Richtung verschiebbar ist, kann insbesondere bei einer Bajonettkupplung eine zuverlässige Verriegelung des Werkzeugs am distalen Ende eines Schafts ermöglicht werden.

Die Verriegelungseinrichtung greift insbesondere in eine Nut bzw. einen Schlitz an der als Bajonettkupplung ausgebildeten Kupplungseinrichtung des Verbindungsbauteil ein.

Bei einem Werkzeug, wie es hier beschrieben ist, umfasst die Verriegelungseinrichtung insbesondere einen ringförmigen Abschnitt, der in eine umlaufende Nut an der Übertragungsstange eingreift.

Bei einem Werkzeug mit einer Übertragungsstange, wie es hier beschrieben ist, weist die Verriegelungseinrichtung zumindest entweder ein elektrisch isolierendes Material auf oder ist gegenüber der Übertragungsstange elektrisch isoliert.

Eine elektrische Isolation der Verriegelungseinrichtung gegenüber der Übertragungsstange oder eine elektrisch isolierende Eigenschaft der Verriegelungseinrichtung selbst ermöglicht eine elektrisch bipolare Ausführung des Werkzeugs, bei dem beispielsweise ein erstes Maulteil über die Übertragungsstange mit einem ersten Pol einer Spannungsquelle und ein zweites Maulteil über die Bajonettkupplung und einen Schaft mit einem zweiten Pol der Spannungsquelle verbindbar sind.

Bei einem Werkzeug, wie es hier beschrieben ist, weist die Gelenkeinrichtung insbesondere ein elektrisch isolierendes Material auf, wobei das Werkzeug ferner ein elektrisch leitfähiges Leiterbauteil umfasst, das zumindest teilweise in der Gelenkeinrichtung angeordnet ist, zur elektrischen Kontaktierung eines Maulteils oder einer anderen Wirkeinrichtung, das bzw. die gegenüber der Übertragungsstange elektrisch isoliert ist.

Das elektrisch leitfähige Leiterbauteil ist insbesondere elastisch ausgebildet, beispielsweise aufgrund eines helikalen Abschnitts. Durch diese Elastizität ermöglicht das elektrisch leitfähige Leiterbauteil eine Kontaktierung eines gegenüber der Gelenkeinrichtung schwenkbaren Maulteils oder einer anderen gegenüber der Gelenkeinrichtung schwenkbaren Wirkeinrichtung. Das elektrische leitfähige Leiterbauteil ist insbesondere mit dem erwähnten Kontaktbauteil elektrisch leitfähig verbunden. Dies ermöglicht eine Zuführung einer Hochspannung und eines entsprechenden Stroms über einen Bajonettverschluss zwischen Schaft und Werkzeug, das Verbindungsbauteil, die Stützhülse, das Kontaktbauteil und das elektrisch leitfähige Bauteil zu einem der Maulteile oder einer anderen Wirkeinrichtung des Werkzeugs. Wenn das zweite oder ein weiteres Maulteil oder eine zweite oder eine weitere Wirkeinrichtung des Werkzeugs über die Übertragungsstange elektrisch kontaktiert ist, kann das Werkzeug als bipolares Werkzeug zur HF-Koagulation verwendet werden.

Die Kupplungseinrichtung ist insbesondere als Bajonettkupplung mit einer Knagge ausgebildet, wobei die Verriegelungseinrichtung einen konvexen Abschnitt umfasst, und wobei die Knagge und der konvexe Abschnitt an der Verriegelungseinrichtung in axialer Richtung und in umfänglicher Richtung beabstandet und ausgebildet sind, um in eine Nut oder einen Schlitz an einem distalen Ende eines Schafts einzugreifen.

Die Bajonettkupplung umfasst insbesondere zwei oder mehr symmetrisch angeordnete Knaggen, die zum Eingriff in jeweils eine Nut oder einen Schlitz an einem distalen Ende eines Schafts ausgebildet sind. Die Knagge oder die Knaggen sind insbesondere jeweils zum Eingriff in eine L-förmige Nut bzw. einen L-förmigen Schlitz mit einem sich in axialer Richtung erstreckenden Abschnitt und einem sich in umfänglicher Richtung erstreckenden Abschnitt ausgebildet. Die Knagge und der konvexe Abschnitt an der Verriegelungseinrichtung - im Falle mehrerer Knaggen und mehrerer konvexer Abschnitte je eine Knagge und ein konvexer Abschnitt - sind dazu ausgebildet und angeordnet, um in ein und dieselbe Nut bzw. ein und denselben Schlitz einzugreifen.

Durch die Rotierbarkeit der Verriegelungseinrichtung gegenüber der Übertragungsstange kann eine Montage und Demontage des Werkzeugs und eines Schafts ähnlich wie bei einer herkömmlichen Bajonettkupplung möglich sein. Dadurch kann eine intuitive Handhabung durch medizinisches Personal ermöglicht oder vereinfacht werden. Zusätzlich ermöglicht die Rotierbarkeit der Übertragungsstange gegenüber der Verriegelungseinrichtung eine Übertragung eines Drehmoments und einer Drehbewegung mittels der Übertragungsstange zum Werkzeug.

Bei einem Werkzeug, wie es hier beschrieben ist, mit einer von einem konvexen Abschnitt der Verriegelungseinrichtung in axialer Richtung und in umfänglicher Richtung beabstandeten Knagge und einer zwischen einer Montageposition und einer Arbeitsposition verschiebbaren Verriegelungseinrichtung ist die Verriegelungseinrichtung insbesondere ausgebildet und angeordnet, um in der Arbeitsposition durch Eingriff in einen sich in axialer Richtung erstreckenden Abschnitt einer Nut oder eines Schlitzes an einem distalen Ende eines Schafts eine Rotation der Kupplungseinrichtung relativ zu dem distalen Ende des Schafts zu hemmen.

Durch die Hemmung der Rotation der Kupplungseinrichtung relativ zum distalen Ende des Schafts wird insbesondere die Knagge der Bajonettkupplung des Werkzeugs in einem sich in umfänglicher Richtung erstreckenden Abschnitt der Nut oder des Schlitzes am distalen Ende des Schafts gehalten.

Die als Bajonettkupplung ausgebildete Kupplungseinrichtung umfasst insbesondere (und insbesondere anstelle einer oder mehrerer Knaggen) eine Nut oder einen Schlitz oder zwei oder mehr Nuten oder Schlitze. Dieser oder diese Nuten oder Schlitze sind insbesondere abgewinkelt mit einem parallel oder im Wesentlichen parallel zur Längsachse sich erstreckenden Abschnitt und einem in umfänglicher Richtung oder im Wesentlichen in umfänglicher Richtung sich erstreckenden Abschnitt.

Die Verriegelungseinrichtung weist insbesondere mehrere Arbeitspositionen bzw. einen Bereich von Arbeitspositionen, der im Folgenden auch als Arbeitsbereich bezeichnet wird, auf. Die Montageposition der Verriegelungseinrichtung liegt insbesondere distal beabstandet von der Arbeitsposition bzw. von dem Arbeitsbereich. Die Verriegelungseinrichtung ist insbesondere linear parallel zur Längsachse des Werkzeugs verschiebbar. Zusätzlich kann die Verriegelungseinrichtung rotierbar sein. Insbesondere ist die Verriegelungseinrichtung zusammen mit dem Verbindungsbauteil relativ zur Gelenkeinrichtung des Werkzeugs rotierbar.

Mit einer in axialer Richtung und damit insbesondere senkrecht zu einem in umfänglicher Richtung sich erstreckenden Abschnitt einer Nut oder eines Schlitzes einer Bajonettkupplung verschiebbaren Verriegelungseinrichtung kann eine zuverlässige Verriegelung des Werkzeugs am distalen Ende eines Schafts ermöglicht werden.

Die Verriegelungseinrichtung greift insbesondere in eine Nut bzw. einen Schlitz an der als Bajonettkupplung ausgebildeten Kupplungseinrichtung des Werkzeugs ein.

Die Nut bzw. der Schlitz ist vorgesehen, um eine Knagge bzw. eine Klaue am distalen Ende eines mit dem Werkzeug zu verbindenden Schafts aufzunehmen. Durch eine insbesondere axiale Verschiebung der Verriegelungseinrichtung innerhalb der Nut bzw. des Schlitzes kann eine mechanische Kopplung zwischen dem Werkzeug und einem distalen Ende eines Schafts verriegelt werden.

Insbesondere weist die Nut bzw. der Schlitz eine T-förmige Gestalt mit einem axialen Abschnitt, der sich in axialer Richtung erstreckt, und einem umfänglichen Abschnitt, der sich in umfänglicher Richtung erstreckt auf, wobei ein proximaler Bereich des axialen Abschnitts proximal einer Mündung des umfänglichen Abschnitts in den axialen Abschnitt angeordnet ist, wobei ein distaler Bereich des axialen Abschnitts distal einer Mündung des umfänglichen Abschnitts in den axialen Abschnitt angeordnet ist, wobei die Verriegelungseinrichtung in der Montageposition nur in den distalen Bereich des axialen Abschnitts der Nut bzw. des Schlitzes eingreift, so dass eine Knagge an einem mit dem Werkzeug zu verbindenden Schaft durch den proximalen Bereich des axialen Abschnitts in den umfänglichen Abschnitt der Nut bzw. des Schlitzes eingeführt werden kann, und wobei die Verriegelungseinrichtung in der Arbeitsposition zumindest teilweise die Mündung des umfänglichen Abschnitts der Nut bzw. des Schlitzes in den axialen Abschnitt blockiert, so dass eine im umfänglichen Abschnitt der Nut bzw. des Schlitzes angeordnete Knagge an einem mit dem Werkzeug verbundenen Schaft durch die Verriegelungseinrichtung im umfänglichen Abschnitt gehalten wird.

Die T-förmige Gestalt der Nut bzw. des Schlitzes umfasst insbesondere auch eine im Wesentlichen T-förmige Gestalt, bei der der axiale Abschnitt sich im Wesentlichen in axialer Richtung erstreckt und/oder der umfängliche Abschnitt sich im Wesentlichen in umfänglicher Richtung erstreckt, wobei die Winkel zwischen dem axialen Abschnitt und dem umfänglichen Abschnitt von 90° abweichen, um beispielsweise innerhalb eines Bereichs von 70° bis 110° oder von 80° bis 100° zu liegen. Ferner können sowohl der axiale Abschnitt als auch der umfängliche Abschnitt der Nut bzw. des Schlitzes jeweils eine gekrümmte Gestalt aufweisen. Beispielsweise kann durch eine bogenförmige Gestalt des axialen Abschnitts und/oder durch eine bogenförmige Gestalt des umfänglichen Abschnitts die Montage und die Demontage vereinfacht werden.

Die beschriebene T-förmige oder im Wesentlichen T-förmige Gestalt der Nut bzw. des Schlitzes, die bzw. der für eine Knagge am distalen Ende eines mit dem Werkzeug mechanisch zu koppelnden Schafts vorgesehen ist, und der Eingriff der Verriegelungseinrichtung in einen axialen Abschnitt kann eine einfache und robuste Verriegelung der mechanischen Kopplung zwischen Werkzeug und Schaft ermöglichen. Konstruktion und Herstellung des Werkzeugs können insbesondere dadurch vereinfacht werden, dass die Nut bzw. der Schlitz gleichzeitig zwei Funktionen erfüllt, nämlich den Formschluss mit einer Knagge am distalen Ende eines Schafts und die Führung der Verriegelungseinrichtung. Die Bajonettkupplung kann so ausgebildet sein, dass sie beispielsweise von medizinischem Personal ohne jede Umgewöhnung genauso gehandhabt werden kann, wie jede herkömmliche Bajonettkupplung.

Bei einem Werkzeug, wie es hier beschrieben ist, ist insbesondere das Maulteil von einem weiteren Maulteil elektrisch isoliert, wobei das weitere Maulteil elektrisch leitfähig mit einem Kontaktbauteil verbunden ist, das mit der Gelenkeinrichtung mechanisch starr verbunden ist, wobei eine proximale Stirnfläche des Kontaktbauteils an einer mit dem Verbindungsbauteil mechanisch starr verbundenen Kontaktfläche anliegt, wobei das Kontaktbauteil und die mit dem Verbindungsbauteil mechanisch starr verbundene Kontaktfläche vorgesehen und ausgebildet sind, um eine elektrisch leitfähige Verbindung zwischen einem mit dem Werkzeug verbundenen Schaft und dem weiteren Maulteil zu bilden.

Das Maulteil und das weitere Maulteil des Werkzeugs sind insbesondere ausgebildet, um mit je einem Pol einer elektrischen Spannungs-, Strom- oder Leistungsquelle verbunden zu werden. Die proximale Stirnfläche des Kontaktbauteils und die mit dem Verbindungsbauteil mechanisch starr verbundene Kontaktfläche sind jeweils insbesondere kreisringförmig. Die proximale Stirnfläche des Kontaktbauteils und die mit dem Verbindungsbauteil mechanisch starr verbundene Kontaktfläche sind jeweils insbesondere eben.

Über die mit dem Verbindungsbauteil mechanisch starr verbundene Kontaktfläche und die proximale Stirnfläche des Kontaktbauteils ist eine elektrisch leitfähige Verbindung des weiteren Maulteils mit dem Schaft unabhängig von der Position des Werkzeug relativ zum Verbindungsbauteil und zum Schaft möglich. Insbesondere eine jeweils kreisringförmige und ebene Ausgestaltung der proximalen Stirnfläche des Kontaktbauteils und der mit dem Verbindungsbauteil mechanisch starr verbundenen Kontaktfläche können einfach und kostengünstig realisierbar sein. Wenn die Maulteile durch eine auf die Übertragungsstange wirkende Zugkraft nach proximal in Richtung auf ihre geschlossenen Positionen bewegt werden, wird die proximale Stirnfläche des Kontaktbauteils gegen die mit dem Verbindungsbauteil mechanisch starr verbundenen Kontaktfläche gedrückt. Deshalb besteht der elektrische Kontakt zwischen dem Kontaktbauteil und der mit dem Verbindungsbauteil mechanisch starr verbundenen Kontaktfläche zumindest dann, wenn die Maulteile gegen einen mechanischen Widerstand geschlossen werden.

Bei einem Werkzeug, wie es hier beschrieben ist, kann das Verbindungsbauteil nahe seinem distalen Ende einen Kragen aufweisen, der von mehreren im Wesentlichen axialen Schlitzen unterbrochen ist und in eine korrespondierende Nut an der Gelenkeinrichtung eingreift, wobei das Werkzeug ferner eine Stützeinrichtung aufweist, die ausgebildet ist, um eine radiale Verformung des Kragens zu hemmen und den Kragen in der Nut zu halten.

Kragen und Nut sind hinsichtlich ihrer Gestalt und ihrer Abmessungen insbesondere so ausgebildet, dass sie eine reibungsarme und spielarme Rotation der Gelenkeinrichtung gegenüber dem Verbindungsbauteil ermöglichen. Der Kragen ragt insbesondere radial nach außen, um von innen in die korrespondierende, nach radial innen offene umlaufende Nut an der Gelenkeinrichtung einzugreifen. Die Stützeinrichtung ist in diesem Fall innerhalb des Verbindungsbauteils angeordnet, insbesondere in radialer Richtung dem Kragen gegenüberliegend. Die Stützeinrichtung ist insbesondere eine Stützhülse in Form eines Rings oder eines Rohres, dessen Außenseite an einer Innenseite des Verbindungsbauteils anliegt und so eine radiale Verformung des Kragens verhindert. Alternativ kann der Kragen am Verbindungsbauteil von außen in eine umlaufende und nach außen offene Nut an der Gelenkeinrichtung eingreifen, wobei die Stützeinrichtung beispielsweise eine, das Verbindungsbauteil nahe dem Kragen umschließende Stützhülse ist.

Ein in eine Nut an der Gelenkeinrichtung eingreifender Kragen am Verbindungsbauteil kann eine formschlüssige, zuverlässige und robuste Verbindung zwischen Gelenkeinrichtung und Verbindungsbauteil ermöglichen. Die axialen Schlitze können eine einfache Fertigung des Werkzeugs ermöglichen, bei der das Verbindungsbauteil im Bereich des Kragens elastisch verformt wird, während es in axialer Richtung in die Gelenkeinrichtung eingeführt oder über die Gelenkeinrichtung gestülpt wird. Danach kann durch die Stützhülse oder eine andere Stützeinrichtung die elastische Verformung des Kragens unterbunden werden, um das Verbindungsbauteil und die Gelenkeinrichtung dauerhaft formschlüssig zu verbinden.

Ein Werkzeug, wie es hier beschrieben ist, bei dem die Stützeinrichtung als Stützhülse ausgebildet ist, kann ferner ein Kontaktbauteil mit einer Kontaktfläche, die an der Stützhülse anliegt, umfassen, wobei das Kontaktbauteil ausgebildet und angeordnet ist, zusammen mit der Gelenkeinrichtung gegenüber dem Verbindungsbauteil rotierbar zu sein.

Das Kontaktbauteil ist beispielsweise hülsenförmig bzw. rohrförmig mit Längsschlitzen ausgebildet. Durch ein leichtes Übermaß wird das Kontaktbauteil von der Stützhülse elastisch verformt, um eine ausreichende Kontaktkraft zwischen dem Kontaktbauteil und der Stützhülse für eine zuverlässige elektrische Kontaktierung zu erzeugen.

Die Stützhülse vereint somit zwei Funktionen, nämlich die des mechanischen Stützens des Kragens am Verbindungsbauteil und die der elektrischen Kontaktierung, wodurch ein besonders einfacher und kompakter Aufbau ermöglicht werden kann.

Bei einem Werkzeug, wie es hier beschrieben ist, ist das Maulteil insbesondere gekrümmt.

Insbesondere umfasst das Werkzeug zwei oder mehr gekrümmte Maulteile. Sofern ein gekrümmtes Maulteil um eine Schwenkachse schwenkbar ist, ist es insbesondere in einer Ebene senkrecht zu der Schwenkachse oder in einer Ebene parallel zur Schwenkachse oder in beiden Richtungen gekrümmt.

Bogenförmig oder sogar schraubenförmig gekrümmte Backen, Schneiden oder andere Maulteile von mikroinvasiv-chirurgischen Instrumenten sind für einige Anwendungen besonders geeignet. Bei der Verwendung eines Werkzeugs mit zwei gekrümmten Maulteilen kann aber im Gegensatz zu einem Werkzeug mit zwei gleichen oder ähnlichen, geraden oder im Wesentlichen geraden Maulteilen eine Rotation um mehr als 90 Grad (bis zu 180 Grad) erforderlich sein, um das Werkzeug relativ zu einem Gegenstand richtig auszurichten. Eine Rotierbarkeit eines Werkzeugs mit einem oder mehreren gekrümmten Maulteilen um eine Längsachse eines Schafts eines Werkzeugs kann deshalb besonders vorteilhaft sein.

Ein mikroinvasiv-chirurgisches Instrument umfasst einen Schaft mit einem proximalen Ende, das mit einer Handhabungseinrichtung koppelbar oder gekoppelt ist, und einem distalen Ende und ein Werkzeug, wie es hier beschrieben ist, das mit dem distalen Ende des Schafts lösbar mechanisch koppelbar ist.

Der Schaft des mikroinvasiv-chirurgischen Instruments kann gerade oder gekrümmt, starr oder flexibel sein. Im Fall eines gekrümmten oder flexiblen Schafts ist insbesondere eine zumindest abschnittsweise biegeflexible Übertragungsstange vorgesehen. Das Werkzeug umfasst insbesondere eine Fass-, Präparier-, Biopsie- oder andere Zange, eine Schere oder einen Nadelhalter mit mindestens zwei geraden oder gekrümmten Backen, Schneiden oder anderen Maulteilen, von denen mindestens eine bzw. eines bewegbar ist. Alternativ umfasst das Werkzeug eine andere Wirkeinrichtung, beispielsweise einen Manipulator mit einem Finger bzw. einer fingerförmigen Einrichtung oder eine Elektrode in Hakenform oder in anderer Gestalt. Die Übertragungsstange ist insbesondere zum Übertragen einer Zug- und/oder Schubkraft zum Werkzeug am distalen Ende des Schafts ausgebildet.

Mit den hier beschriebenen Merkmalen und Eigenschaften des Werkzeugs und entsprechenden Merkmalen und Eigenschaften des Schafts und der Übertragungsstange sind eine einfache, schnelle und zuverlässige Montage und effiziente Verwendung des mikroinvasiv-chirurgischen Instruments möglich.

Insbesondere ist die Übertragungsstange zur Übertragung eines Drehmoments von der Handhabungseinrichtung zu einem Werkzeug am distalen Ende des Schafts ausgebildet. Die Übertragungsstange ist somit insbesondere hinsichtlich Druck- und Zugbelastungen in Längsrichtung und hinsichtlich Torsion steif bzw. unelastisch. Gleichzeitig kann die Übertragungsstange biegeflexibel sein, insbesondere im Fall eines zumindest abschnittsweise gekrümmten Schafts.

Die lösbare Verbindung zwischen Werkzeug und Schaft kann eine Reinigung des Instruments verbessern oder überhaupt erst ermöglichen. Die Verriegelung des Werkzeugs am distalen Ende des Schafts, wenn die Position der mit dem proximalen Ende der Übertragungsstange gekoppelten Stangenkupplung in dem vorbestimmten Arbeitsbereich liegt, kann eine vollständige Zerlegung des Instruments allein aufgrund eines Lösens der Arretierung des Schafts an der Handhabungseinrichtung ermöglichen. Dies kann die Handhabung des Instruments, insbesondere sein Zerlegen und Zusammensetzen deutlich vereinfachen oder verbessern.

Bei einem mikroinvasiv-chirurgischen Instrument, wie es hier beschrieben ist, kann das distale Ende des Schafts einen Bajonettkupplungsbereich mit einer L-förmigen Nut oder einem L-förmigen Schlitz aufweisen.

Die L-förmige Nut bzw. der L-förmige Schlitz umfasst insbesondere einen sich in axialer Richtung erstreckenden Abschnitt und einen sich in umfänglicher Richtung erstreckenden Abschnitt.

### Kurzbeschreibung der Figuren

Nachfolgend werden Ausführungsformen anhand der beigefügten Figuren näher erläutert. Es zeigen:
- Figur 1: eine schematische Darstellung eines mikroinvasiv-chirurgischen Instruments;
- Figur 2: eine schematische Darstellung des mikroinvasiv-chirurgischen Instruments aus Figur 1 in zerlegter Form;
- Figur 3: eine schematische Darstellung eines Werkzeugs für ein mikroinvasiv-chirurgisches Instrument;
- Figur 4: eine weitere schematische Darstellung des Werkzeugs aus Figur 3;
- Figur 5: eine weitere schematische Darstellung des Werkzeugs aus den Figuren 3 und 4;
- Figur 6: eine schematische Darstellung eines weiteren Werkzeugs für ein mikroinvasiv-chirurgisches Instrument;
- Figur 7: eine weitere schematische Darstellung des Werkzeugs aus Figur 6;
- Figur 8: eine weitere schematische Darstellung des Werkzeugs aus den Figuren 6 und 7;
- Figur 9: eine weitere schematische Darstellung des Werkzeugs aus den Figuren 6 bis 8;
- Figur 10: eine schematische Darstellung eines weiteren Werkzeugs für ein mikroinvasiv-chirurgisches Instrument;
- Figur 11: eine weitere schematische Darstellung des Werkzeugs aus Figur 10;
- Figur 12: eine weitere schematische Darstellung des Werkzeugs aus den Figuren 10 und 11;
- Figur 13: eine schematische Darstellung von Kupplungseinrichtungen;
- Figur 14: eine weitere schematische Darstellung der Kupplungseinrichtungen aus Figur 13;
- Figur 15: eine weitere schematische Darstellung der Kupplungseinrichtungen aus den Figuren 13 und 14;
- Figur 16: eine weitere schematische Darstellung der Kupplungseinrichtungen aus den Figuren 13 bis 15.
- Figur 17: eine weitere schematische Darstellung des Werkzeugs aus den Figuren 10 bis 12;
- Figur 18: eine weitere schematische Darstellung des Werkzeugs aus den Figuren 10 bis 12 und 17;
- Figur 19: eine schematische Darstellung eines weiteren Werkzeugs für ein mikroinvasiv-chirurgisches Instrument;
- Figur 20: eine weitere schematische Darstellung des Werkzeugs aus Figur 19;
- Figur 21: eine weitere schematische Darstellung des Werkzeugs aus den Figuren 19 und 20;
- Figur 22: eine schematische Darstellung von Kupplungseinrichtungen;
- Figur 23: eine weitere schematische Darstellung der Kupplungseinrichtungen aus Figur 22;
- Figur 24: eine weitere schematische Darstellung der Kupplungseinrichtungen aus den Figuren 22 und 23;
- Figur 25: eine weitere schematische Darstellung der Kupplungseinrichtungen aus den Figuren 22 bis 24.
- Figur 26: eine weitere schematische Darstellung des Werkzeugs aus den Figuren 19 bis 21;
- Figur 27: eine weitere schematische Darstellung des Werkzeugs aus den Figuren 19 bis 21 und 26.

### Beschreibung der Ausführungsformen

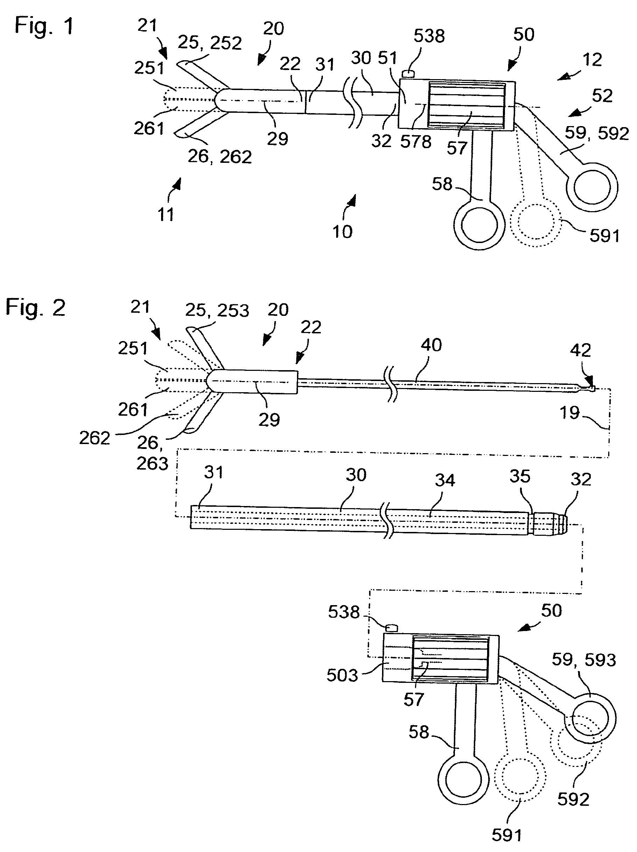

Figur 1 zeigt eine schematische Darstellung eines mikroinvasiv-chirurgischen Instruments 10 mit einem distalen Ende 11 und einem proximalen Ende 12. Das mikroinvasiv-chirurgische Instrument 10 umfasst ein Werkzeug 20, einen Schaft 30 und eine Handhabungseinrichtung 50. Am distalen Ende 21 weist das Werkzeug 20 ein erstes bewegbares Maulteil 25 und ein zweites bewegbares Maulteil 26 auf. Die Maulteile 25, 26 sind in Figur 1 in durchgezogenen Linien in offenen Positionen 252, 262 und in gestrichelten Linien in geschlossenen Positionen 251, 261 dargestellt. Die Maulteile 25, 26 können jeweils gerade oder im Wesentlichen gerade oder in Richtung senkrecht zur Zeichenebene der Figur 1 und/oder - abweichend von der Darstellung in Figur 1 - in der Zeichenebene der Figur 1 gekrümmt sein.

Das proximale Ende 22 des Werkzeugs 20 ist lösbar mechanisch gekoppelt mit einem distalen Ende 31 des Schafts 30. Der Schaft 30 ist in Figur 1 stark verkürzt und der Einfachheit halber gerade dargestellt. Abweichend von der Darstellung in Figur 1 kann der Schaft 30 eben oder räumlich gekrümmt sein. Mit einer innerhalb einer Ebene oder - für einige Anwendungen noch vorteilhafter- räumlich gekrümmten Gestalt des Schafts 30 kann das mikroinvasiv-chirurgische Instrument 10 besonders für mikroinvasiv-chirurgische Eingriffe geeignet sein, bei denen ein Endoskop und ein oder mehrere Instrumente gleichzeitig durch einen einzigen Zugang in eine Körperhöhle eingeführt werden.

Das proximale Ende 32 des Schafts 30 ist mit dem distalen Ende 51 der Handhabungseinrichtung 50 lösbar mechanisch gekoppelt. Zur Handhabung des mikroinvasiv-chirurgischen Instruments 10 weist die Handhabungseinrichtung 50 ein Drehrad 57, ein erstes Griffteil 58 und ein zweites Griffteil 59 auf. Das Drehrad 57 ist zur Steuerung einer Rotation des Werkzeugs 20, insbesondere der Maulteile 25, 26, um eine Längsachse 29 vorgesehen. Bei dem in Figur 1 dargestellten Beispiel ist das Drehrad 57 um eine Achse 578 drehbar, die gleichzeitig die Längsachse des Schafts 30 an seinem proximalen Ende 32 ist. Alternativ kann die Achse 578 parallel zur Längsachse des Schafts 30 an seinem proximalen Ende 32 sein. Ferner weist das Drehrad 57 eine Oberflächenstruktur auf, die auch mit Handschuhen eine zuverlässige Bedienung bzw. Betätigung ermöglicht, beispielsweise die angedeuteten Stege in axialer Richtung. Die Griffteile 58, 59 sind insbesondere - abweichend von der in Figur 1 dargestellten stark stilisierten Gestalt - so angeordnet und geformt, dass medizinisches Personal mit einer Hand beide Griffteile 58, 59 ermüdungsarm greifen und relativ zueinander bewegen kann.

Zumindest eines der beiden Griffteile 58, 59 ist relativ zu den anderen Bestandteilen der Handhabungseinrichtung 50 bewegbar. Bei dem in Figur 1 dargestellten Beispiel sind das erste Griffteil 58 starr und das zweite Griffteil 59 bewegbar angeordnet. Das zweite Griffteil 59 ist insbesondere zwischen einer ersten, in Figur 1 in gestrichelter Linie dargestellten Arbeitsposition 591 und einer zweiten, in Figur 1 in durchgezogener Linie dargestellten Arbeitsposition 592 bewegbar. Das zweite Griffteil 59 der Handhabungseinrichtung 50 ist derart mit den Maulteilen 25, 26 des Werkzeugs 20 mechanisch gekoppelt, dass die Maulteile 25, 26 sich in ihren geschlossenen Positionen 251, 261 befinden, wenn das zweite Griffteil 59 seine erste Arbeitsposition 591 einnimmt, und dass die Maulteile 25, 26 sich in ihren offenen Positionen 252, 262 befinden, wenn das zweite Griffteil 59 seine zweite Arbeitsposition 592 einnimmt.

Figur 2 zeigt eine schematische Darstellung von Bestandteilen bzw. Komponenten des oben anhand der Figur 1 dargestellten mikroinvasiv-chirurgischen Instruments 10, die ohne Verwendung von Werkzeug montiert bzw. zum Instrument zusammengesetzt werden können. Ebenso kann das mikroinvasiv-chirurgische Instrument 10 ohne Werkzeug in die in Figur 2 separat dargestellten Bestandteile bzw. Komponenten zerlegt werden. Durch die die gesamte Figur 2 durchlaufende strichpunktierte Linie 19 ist angedeutet, wie diese Bestandteile bzw. Komponenten zusammenzusetzen sind.

Das Werkzeug 20 ist insbesondere dauerhaft mit einer Übertragungsstange 40 verbunden, die zum Übertragen einer Kraft und eines Drehmoments von der Handhabungseinrichtung 50 zum Werkzeug 20 vorgesehen ist. Das in Figur 2 nicht dargestellte distale Ende der Übertragungsstange 40 ist derart mit den Maulteilen 25, 26 gekoppelt, dass eine Bewegung der Übertragungsstange 40 parallel zur Längsachse 29 des Werkzeugs 20 eine synchrone Bewegung der Maulteile 25, 26 bewirkt.

Am proximalen Ende 22 des Werkzeugs 20 und am distalen Ende 31 des Schafts 30 sind in Figur 2 nicht dargestellte Bajonettkupplungseinrichtungen sowie eine mit der Übertragungsstange 40 gekoppelte Verriegelungseinrichtung vorgesehen. Die Maulteile 25, 26 sind in Figur 2 in durchgezogenen Linien in überoffenen Positionen 253, 263 und in gestrichelten Linien in den bereits oben anhand der Figur 1 beschriebenen geschlossenen und offenen Positionen 251, 252, 261, 262 dargestellt. Wenn die Maulteile 25, 26 sich in den überoffenen Positionen 253, 263 befinden, ist die mit den Maulteilen 25, 26 und dem distalen Ende der Übertragungsstange 40 gekoppelte und in Figur 2 nicht dargestellte Verriegelungseinrichtung inaktiv. In diesem Zustand können die Übertragungsstange 40 in einen für die Übertragungsstange 40 vorgesehenen Kanal 34 im Schaft 30 eingeführt und das proximale Ende 22 des Werkzeugs und das distale Ende 31 des Schafts über die in Figur 2 nicht dargestellten Bajonettkupplungseinrichtungen miteinander lösbar mechanisch verbunden bzw. gekoppelt werden. Ferner kann in diesem entriegelten Zustand eine mechanische Kopplung des proximalen Endes 22 des Werkzeugs 20 und des distalen Endes 31 des Schafts 30 über die in Figur 2 nicht dargestellten Bajonettkupplungseinrichtungen gelöst werden.

Wenn die Maulteile 25, 26 sich in den geschlossenen Positionen 251, 261, in den offenen Positionen 252, 262 oder in dazwischen liegenden Positionen befinden, befindet sich die mit dem distalen Ende der Übertragungsstange 40 und mittelbar mit den Maulteilen 25, 26 gekoppelte Verriegelungseinrichtung in einer Arbeitsposition bzw. in einer Position innerhalb eines Arbeitsbereichs. In der Arbeitsposition bzw. in den Positionen innerhalb des Arbeitsbereichs ist die mechanische Kopplung des proximalen Endes 22 des Werkzeugs 20 mit dem distalen Ende 31 des Schafts 30 über die in Figur 2 nicht dargestellten Bajonettkupplungseinrichtungen verriegelt. Wenn die mechanische Verbindung bzw. Kopplung von Werkzeug 20 und Schaft 30 verriegelt ist, können das Werkzeug 20 und der Schaft 30 nicht oder nicht ohne Weiteres zerstörungsfrei voneinander getrennt werden.

Anstelle der Bajonettkupplungseinrichtungen können das proximale Ende 22 des Werkzeugs 20 und das distale Ende 31 des Schafts 30 andere Kupplungseinrichtungen aufweisen. Auch in diesem Fall ist eine Verriegelungseinrichtung am Werkzeug 20 vorgesehen, die die mechanische Verbindung von Werkzeug 20 und Schaft 30 entriegelt, wenn die Maulteile 25, 26 sich in den überoffenen Positionen 253, 263 befinden.

Wenn die Übertragungsstange 40 in den Kanal 34 des Schafts 30 eingesetzt und das proximale Ende 22 des Werkzeugs 20 mit dem distalen Ende 31 des Schafts 30 mechanisch verbunden bzw. gekoppelt ist, kann das proximale Ende 32 des Schafts 30 mit dem gegenüber dem proximalen Ende 32 des Schafts 30 überstehenden proximalen Ende 42 der Übertragungsstange 40 in die Handhabungseinrichtung 50 eingesetzt werden. Dazu weist die Handhabungseinrichtung 50 eine in Figur 2 durch eine punktierte Linie angedeutete Ausnehmung 503 auf.

Zum Einsetzen des proximalen Endes 32 des Schafts 30 und des proximalen Endes 42 der Übertragungsstange 40 in die Handhabungseinrichtung 50 wird das zweite Griffteil 59 zunächst in eine in Figur 2 in durchgezogener Linie dargestellte Koppelposition 593 gebracht. Wenn sich das zweite Griffteil 59 in der Koppelposition 593 befindet, befindet sich eine in Figur 2 nicht dargestellte Stangenkupplung im Inneren der Handhabungseinrichtung 50 in einer Koppelposition, in der sie das proximale Ende 42 der Übertragungsstange 40 aufnehmen oder freigeben kann. Wenn das proximale Ende 42 der Übertragungsstange 40 ganz in die Handhabungseinrichtung 50 eingeführt sind, wird die in Figur 2 nicht dargestellte Stangenkupplung im Inneren der Handhabungseinrichtung 50 mit dem proximalen Ende 42 der Übertragungsstange 40 mechanisch verbunden bzw. gekoppelt. Dabei geht das zweite Griffteil 59 abhängig von den Positionen der Maulteile 25, 26 (geschlossene Positionen 251, 261, offene Positionen 252, 262 oder dazwischen) in die erste Arbeitsposition 591, die zweite Arbeitsposition 592 oder eine Position zwischen der ersten Arbeitsposition 591 und der zweiten Arbeitsposition 592 über.

Wenn das proximale Ende 32 des Schafts 30 ganz in die Handhabungseinrichtung 50 eingeführt ist, greift ein in Figur 2 nicht dargestellter Riegel in eine umlaufende Nut 35 nahe dem proximalen Ende 32 des Schafts 30 ein und verriegelt damit das proximale Ende 32 des Schafts 30 in einer vorgesehenen Position in der Handhabungseinrichtung 50. Durch die Verriegelung des proximalen Endes 32 des Schafts 30 in der Handhabungseinrichtung 50 wird mittelbar auch die mechanische Kopplung des proximalen Endes 42 der Übertragungsstange 40 mit der in Figur 2 nicht dargestellten Stangenkupplung im Innern der Handhabungseinrichtung 50 verriegelt.

Nach Verriegelung des proximalen Endes 32 des Schafts 30 in der Handhabungseinrichtung 50 und mittelbar des proximalen Endes 42 der Übertragungsstange 40 in der in Figur 2 nicht dargestellten Stangenkupplung in der Handhabungseinrichtung 50 ist das mikroinvasiv-chirurgische Instrument 10 wie in Figur 1 dargestellt konfiguriert. Durch Bewegung des zweiten Griffteils 59 relativ zum ersten Griffteil 58 zwischen den beiden Arbeitspositionen 591, 592 können die Maulteile 25, 26 zwischen den geschlossenen Positionen 251, 261 und den offenen Positionen 252, 262 bewegt werden. Durch Rotation des Drehrads 57 um die Achse 578 können die Maulteile 25, 26, um die Längsachse 29 des Werkzeugs 20 gedreht werden.

Abweichend von den Darstellungen in den Figuren 1 und 2 kann der Schaft 30 nahe seinem proximalen Ende 32 ein weiteres Drehrad aufweisen, das nahe dem distalen Ende 51 der Handhabungseinrichtung 50 angeordnet ist, wenn das proximale Ende 32 des Schafts 30 in die Handhabungseinrichtung 50 eingesetzt ist. Mittels dieses in den Figuren 1 und 2 nicht dargestellten Drehrads kann der Schaft 30 um die Längsachse des proximalen Endes 20 des Schafts 30 gedreht werden. Dies ist insbesondere von Bedeutung, wenn der Schaft 30 abweichend von den Darstellungen in den Figuren 1 und 2 gekrümmt ist. In diesem Fall können der gekrümmte Schaft 30 und das Werkzeug 20 am distalen Ende 31 des gekrümmten Schafts 30 unabhängig voneinander gedreht werden.

Durch Druck auf den Entriegelungsknopf 538 kann der in Figur 2 nicht dargestellte Riegel gegen die Kraft einer Feder verschoben und aus der Nut 35 am Schaft 30 ausgerückt werden. Danach kann das proximale Ende 32 des Schafts 30 aus der Handhabungseinrichtung 50 entnommen werden. Dabei wird auch die Verriegelung des proximalen Endes 42 der Übertragungsstange 40 an der in den Figuren 1 und 2 nicht dargestellten Stangenkupplung in der Handhabungseinrichtung 50 gelöst.

Anstelle eines oder - wie in den Figuren 1 und 2 dargestellt - zweier bewegbarer Maulteile 25, 26 kann das Werkzeug 20 eine andere Wirkeinrichtung aufweisen, insbesondere einen Manipulator, beispielsweise einen fingerförmigen Manipulator, oder eine Elektrode, beispielsweise eine hakenförmige Elektrode.

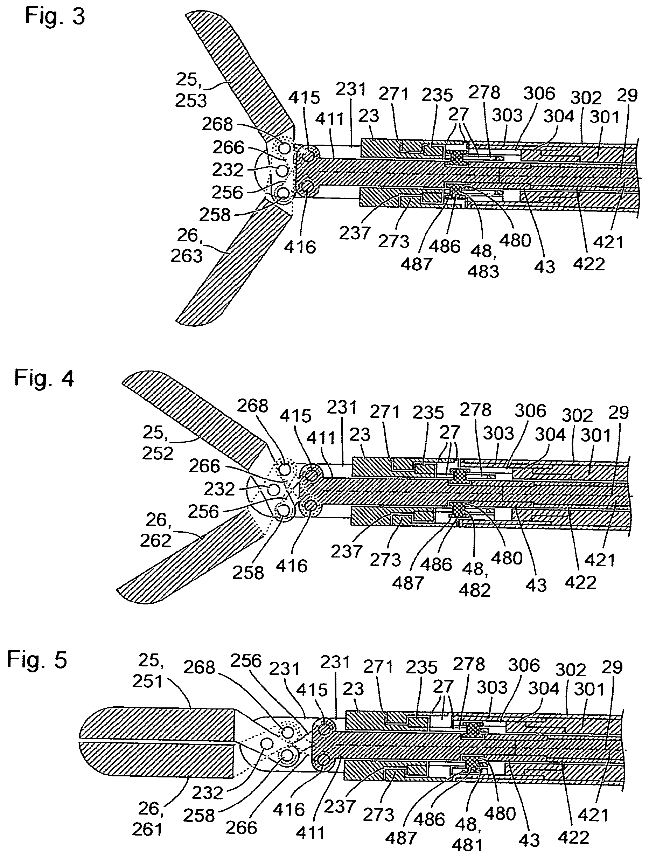

Die Figuren 3 bis 5 zeigen schematische Schnittdarstellungen eines Ausführungsbeispiels des oben anhand der Figuren 1 und 2 dargestellten Werkzeugs 20. Die Schnittebenen der Figuren 3 bis 5 sind parallel zu den Zeichenebenen der Figuren 1 und 2 und enthalten die in den Figuren 1 und 2 angedeutete Längsachse 29 des Werkzeugs 20. Zusätzlich zum Werkzeug 20 ist in jeder der Figuren 3 bis 5 das distale Ende 31 des Schafts 30 gezeigt. In Figur 3 sind die Maulteile 25, 26 in ihren überoffenen Positionen 253, 263 dargestellt, bei denen die mechanische Verbindung zwischen Werkzeug 20 und Schaft 30 entriegelt ist, d.h. hergestellt oder gelöst werden kann. In Figur 4 sind die Maulteile 25, 26 in ihren offenen Positionen 252, 262 dargestellt, die Verbindung zwischen Werkzeug 20 und Schaft 30 ist verriegelt. In Figur 5 sind die Maulteile 25, 26 in ihren geschlossenen Positionen 251, 261 dargestellt, die Verbindung zwischen Werkzeug 20 und Schaft 30 ist ebenfalls verriegelt.

Das Werkzeug weist eine Gelenkeinrichtung 23 auf, die in einem distalen Bereich gabelförmig mit zwei Holmen ausgebildet ist. Ein Holm 231 liegt hinter den Schnittebenen der Figuren 3 bis 5 und ist in den Figuren 3 bis 5 erkennbar. Ein zweiter Holm ist bezüglich den Schnittebenen der Figuren 3 bis 5 symmetrisch zu dem in den Figuren 3 bis 5 gezeigten Holm 231 ausgebildet und angeordnet. Der zweite Holm liegt vor der Schnittebene der Figuren 3 bis 5 und ist deshalb in den Figuren 3 bis 5 nicht dargestellt.

Das Werkzeug 20 umfasst ferner ein Verbindungsbauteil 27, das eine näherungsweise hülsenförmige Gestalt mit zwei jeweils im Wesentlichen zylindermantelförmigen Abschnitten mit unterschiedlichen Durchmessern aufweist. Das Verbindungsbauteil 27 kann, wie in den Figuren 3 bis 5 angedeutet, zwei miteinander gefügte Elemente umfassen. Nahe seinem distalen Ende 271 weist das Verbindungsbauteil 27 einen radial nach innen vorstehenden Kragen 273 auf, der in eine Nut 237 an der Gelenkeinrichtung 23 eingreift. Bei dem in den Figuren 3 bis 5 dargestellten Beispiel ist die Nut 237 an der Gelenkeinrichtung 23 nach proximal aus Fertigungsgründen durch einen Ring 235 begrenzt, der mit der Gelenkeinrichtung 23 gefügt wird, nachdem das Verbindungsbauteil 27 wie vorgesehen angeordnet ist.

Der Kragen 273 an dem Verbindungsbauteil 27 und die umlaufende Nut 237 an der Gelenkeinrichtung 23 bilden eine formschlüssige, spielarme und reibungsarme permanente mechanische Verbindung zwischen der Gelenkeinrichtung 23 und dem Verbindungsbauteil 27. Die durch den Kragen 273 am Verbindungsbauteil und die Nut 237 an der Gelenkeinrichtung 23 gebildete mechanische Verbindung zwischen Verbindungsbauteil 27 und Gelenkeinrichtung 23 erlaubt eine freie Rotation der Gelenkeinrichtung 23 relativ zum Verbindungsbauteil 27 um die Längsachse 29 des Werkzeugs 20. Gleichzeitig unterbindet die durch den Kragen 273 am Verbindungsbauteil und die Nut 237 an der Gelenkeinrichtung 23 gebildete mechanische Verbindung relative axiale Verschiebung parallel zur Längsachse 29 des Werkzeugs 20 und keine relative Kippbewegungen um Achsen senkrecht zur Längsachse 29 des Werkzeugs 20.

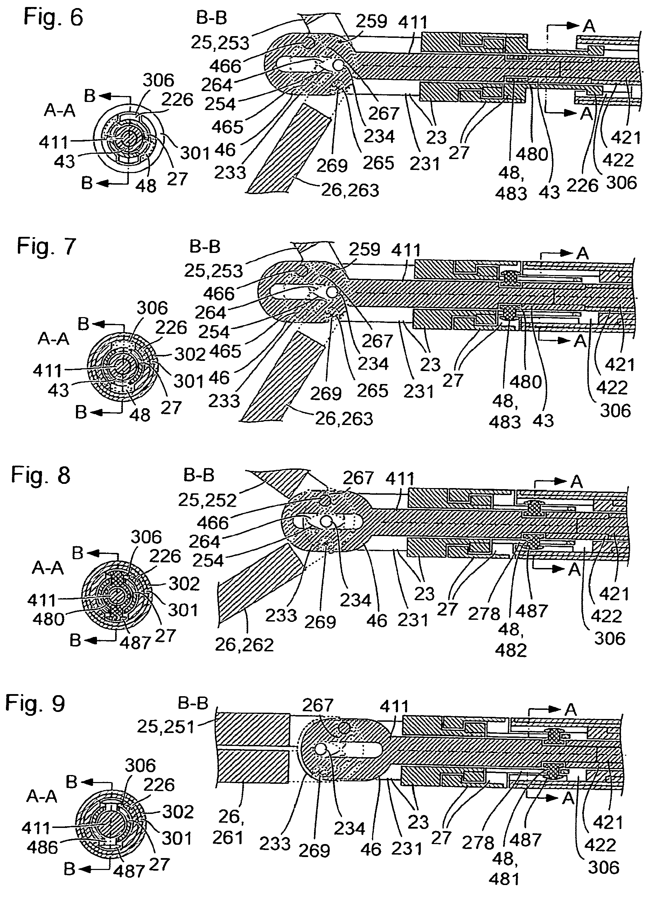

In einem proximalen Bereich ist das Verbindungsbauteil 27 im Wesentlichen rohrförmig bzw. kreiszylindermantelförmig mit einem reduzierten Durchmesser und axialen bzw. sich parallel zur Längsachse 29 des Werkzeugs 20 erstreckenden Schlitzen 278. Die axialen Schlitze 278 bilden Führungen für eine Verriegelungseinrichtung 48, die unten näher beschrieben wird. Ferner sind am proximalen Rand des Verbindungsbauteils 27 Knaggen angeordnet, die in den Figuren 3 bis 5 nicht dargestellt sind, da sie außerhalb der dargestellten Schnittebene liegen. Gestalt und Funktion der Knaggen werden unten mit Bezug auf die Figuren 6 bis 9 beschrieben.

In den Figuren 3 bis 5 ist ferner das distale Ende eines Schafts 30 erkennbar, der mit dem Werkzeug 20 mechanisch verbunden bzw. gekoppelt ist. Der Schaft 30 umfasst ein Metallrohr 301, an dessen distales Ende eine Bajonetthülse 304 und ein die Bajonetthülse 304 zylindermantelförmig umschließendes Stützrohr 303 gefügt sind. Die Bajonetthülse 304 weist von ihrem distalen Rand ausgehende L-förmige Schlitze 306 mit je einem in axialer Richtung und einem in umfänglicher Richtung sich erstreckenden Abschnitt auf. Das Metallrohr 301 und das Stützrohr 303 sind von einem Isoliermantel 302 aus einem elektrisch isolierenden Material mantelförmig umgeben.

Die in den Figuren 3 bis 5 nicht gezeigten Knaggen und die L-förmigen Schlitze 306 in den Bajonetthülsen 304 am distalen Ende des Schafts 30 sind angeordnet und ausgebildet, um korrespondierende Bajonettkupplungseinrichtungen zur lösbaren mechanischen Kopplung bzw. Verbindung des Werkzeugs 20 und des Schafts 30 zu bilden. Die Funktionsweise dieser Bajonettkupplung ist unten mit Bezug auf die Figuren 6 bis 9 näher beschrieben.

Die bereits in Figur 2 angedeutete Übertragungsstange 40 umfasst einen distalen Abschnitt 411 und einen proximalen Abschnitt 421, die mittels einer Verbindungshülse 43 mechanisch starr miteinander verbunden sind.

Am äußersten distalen Ende weist der distale Abschnitt 411 der Übertragungsstange 40 zwei Gelenke 415, 416 auf. Ein erstes Pleuel 256 verbindet das erste Gelenk 415 am distalen Ende der Übertragungsstange 40 mit einem von der Welle 232 beabstandeten Gelenk 258 am ersten Maulteil 25. Ein zweites Pleuel 266 verbindet das zweite Gelenk 416 am distalen Ende der Übertragungsstange 40 mit einem von der Welle 232 beabstandeten Gelenk 268 am zweiten Maulteil 26. In der Zusammenschau der Figuren 3 bis 5 ist erkennbar, dass eine lineare Verschiebung der Übertragungsstange 40 parallel zur Längsachse 29 des Werkzeugs 20 vermittels der Pleuel 256, 266 ein Schwenken der Maulteile 25, 26 um die durch die Welle 232 gebildeten Gelenke bewirkt.

In einem proximalen Bereich weist der distale Abschnitt 411 der Übertragungsstange 40 einen stufenartig reduzierten Querschnitt auf. An dieser Stelle bildet der distale Abschnitt 411 der Übertragungsstange 40 zusammen mit der Verbindungshülse 43 eine ringförmig umlaufende Nut, in der ein ringförmiger Abschnitt 480 einer Verriegelungseinrichtung 48 angeordnet ist. Der ringförmige Abschnitt 480 ist in der durch den distalen Abschnitt 411 der Übertragungsstange 40 und die Verbindungshülse 43 gebildeten umlaufenden Nut spielarm und reibungsarm geführt. Durch die spiel- und reibungsarme Führung des ringförmigen Abschnitts 480 an der Übertragungsstange 40 ist die Verriegelungseinrichtung 48 hinsichtlich einer axialen Bewegung parallel zur Längsachse 29 des Werkzeugs 20 von einem geringen axialen Spiel abgesehen starr mit der Übertragungsstange 40 verbunden. Gleichzeitig ist die Verriegelungseinrichtung 48 um die Längsachse 29 des Werkzeugs 20 relativ zur Übertragungsstange 40 reibungsarm rotierbar.

Die Verriegelungseinrichtung 48 weist radial nach außen ragende Zapfen 486 auf, die durch jeweils einen der axialen Schlitze 278 des Verbindungsbauteils 27 hindurch reichen. Radial außerhalb der axialen Schlitze 278 weist die Verriegelungseinrichtung 48 je einen ringförmigen Kopf 487 auf, der an das radial äußere Ende je eines Zapfens 486 gefügt ist. Die radialen Zapfen 486 an der Verriegelungseinrichtung 48 und die axialen Schlitze 278 am Verbindungsbauteil 27 sind so ausgebildet und angeordnet, dass das Verbindungsbauteil 48 in axialer Richtung parallel zur Längsachse 29 des Werkzeugs 20 innerhalb eines vorbestimmten Bereichs frei relativ zum Verbindungsbauteil 27 reibungsarm verschoben werden kann und gleichzeitig hinsichtlich einer Rotation um die Längsachse 29 des Werkzeugs 20 durch die Schlitze 278 spielarm geführt ist.

Eine axiale Verschiebung der Übertragungsstange 40 parallel zur Achse 29 des Werkzeugs 20, wie sie im Vergleich der Figuren 3 bis 5 erkennbar ist, hat somit eine entsprechende Verschiebung der Verriegelungseinrichtung 48 zur Folge. Insbesondere befindet sich die Verriegelungseinrichtung 48 in der in Figur 3 dargestellten Montageposition 483, wenn sich die Maulteile 25, 26 in ihren in Figur 2 gezeigten überoffenen Positionen 253, 263 befinden. Die Verriegelungseinrichtung 48 befindet sich in der in Figur 5 dargestellten ersten Arbeitsposition 481, wenn sich die Maulteile 25, 26 in ihren geschlossenen Positionen 251, 261 befinden. Die Verriegelungseinrichtung 48 befindet sich in der in Figur 4 dargestellten zweiten Arbeitsposition 482, wenn sich die Maulteile 25, 26 in ihren offenen Positionen 252, 262 befinden. In allen Positionen 481, 482, 483 der Verriegelungseinrichtung 48 können die Übertragungsstange 40 und mit ihr die Gelenkeinrichtung 23 und die Maulteile 25, 26 relativ zum Verbindungsbauteil 27 und zum Schaft 30 frei um die Längsachse 29 des Werkzeugs 20 rotiert werden.

Bei der in Figur 3 gezeigten Montageposition 483 der Verriegelungseinrichtung 48, greift die Verriegelungseinrichtung 48 nicht in den L-förmigen Schlitz 306 in der Bajonetthülse 304 am distalen Ende des Schafts 30 ein. Deshalb können in der Montageposition 483 der Verriegelungseinrichtung 48 das Verbindungsbauteil 27 und der Schaft 30 relativ zueinander gedreht werden, um die in den Figuren 3 bis 5 nicht gezeigte Knagge in den L-förmigen Schlitz 306 in der Bajonetthülse 304 einzuführen und aus dieser zu entnehmen.

Wie erwähnt sind in Figur 5 die Verriegelungseinrichtung 48 in einer ersten Arbeitsposition 481 und die Maulteile 25, 26 in ihren geschlossenen Positionen 251, 261 gezeigt. In Figur 4 sind die Verriegelungseinrichtung 48 in einer zweiten Arbeitsposition und die Maulteile 25, 26 in ihren offenen Positionen 252, 262 gezeigt. In beiden Arbeitspositionen 481, 482 greift die Verriegelungseinrichtung 48 in axiale Abschnitte der L-förmigen Schlitze 306 in der Bajonetthülse 304 am distalen Ende des Schafts 30 ein. Insbesondere greifen in beiden Arbeitspositionen 481, 482 der Verriegelungseinrichtung 48 die radial außerhalb der axialen Schlitze 278 des Verbindungsbauteils 27 angeordneten Köpfe 487 in die axialen Abschnitte der L-förmigen Schlitze 306 in der Bajonetthülse 304 am distalen Ende des Schafts 30 ein. Dadurch können das Verbindungsbauteil 27 und der Schaft 30 nicht relativ zueinander gedreht werden. Die in den Figuren 3 bis 5 nicht gezeigten Knaggen am proximalen Rand des Verbindungsbauteils 27 greifen in die umfänglichen Abschnitte der L-förmigen Schlitze 306 in der Bajonetthülse 304 ein, so dass das Verbindungsbauteil 27 und damit das gesamte Werkzeug 20 mit dem Schaft 30 formschlüssig verbunden bzw. gekoppelt sind. Anstelle der Köpfe 487 kann die Verriegelungseinrichtung 48 einen oder mehrere anders ausgestaltete konvexe Abschnitte aufweisen, die in die axialen Abschnitte der L-förmigen Schlitze 306 in der Bajonetthülse 304 eingreifen.

Die Figuren 6 bis 9 zeigen schematische Darstellungen eines weiteren Ausführungsbeispiels des Werkzeugs 20 des mikroinvasiv-chirurgischen Instruments 10 aus den Figuren 1 und 2.

Jede der Figuren 6 bis 9 zeigt einen Schnitt entlang einer Schnittebene B-B parallel zur Längsachse 29 des Werkzeugs 20 und einen Schnitt entlang einer Schnittebene A-A senkrecht zur Längsachse 29 des Werkzeugs 20. Die Schnittebene B-B enthält die Längsachse 29 des Werkzeugs 20 und entspricht den Schnittebenen der Figuren 3 bis 5. Die Lage der Schnittebene A-A ist in der Darstellung des Schnitts entlang der Ebene B-B angedeutet. Die Lage der Schnittebene B-B ist in der Darstellung des Schnitts entlang der Ebene A-A angedeutet.

Das in den Figuren 6 bis 9 gezeigte Werkzeug 20 unterscheidet sich von dem Ausführungsbeispiel der Figuren 3 bis 5 insbesondere durch eine andere mechanische Kopplung zwischen der Übertragungsstange 40 und den Maulteilen 25, 26 zur Übertragung einer linearen Bewegung der Übertragungsstange 40 in Schwenkbewegungen der Maulteile 25, 26. Die mechanische Verbindung bzw. Kopplung zwischen dem Werkzeug 20 und dem distalen Ende 31 eines Schafts 30 ähnelt weitgehend der mechanischen Kopplung bei dem oben anhand der Figuren 3 bis 5 dargestellten Ausführungsbeispiel. Die nachfolgend anhand der Figuren 6 bis 9 beschriebene mechanische Verbindung zwischen dem Werkzeug 20 und dem Schaft 30 und die Verriegelung dieser Verbindung entsprechen deshalb denen für das Ausführungsbeispiel der Figuren 3 bis 5.

Die bei dem in den Figuren 6 bis 9 gezeigtem Werkzeug vorliegende mechanische Kopplung zwischen der Übertragungsstange 40 und dem Maulteilen 25, 26 bedingt bei gleichen Schwenkwinkeln der Maulteile 25, 26 eine lineare Verschiebung der Übertragungsstange 40 in einem größeren Bereich. Entsprechend sind die axialen Schlitze 278 am Verbindungsbauteil 27 bei dem in den Figuren 6 bis 9 gezeigten Werkzeug 20 länger als bei dem Ausführungsbeispiel der Figuren 3 bis 5.

Abweichend von einer reinen Schnittdarstellung ist in den Schnitten entlang der Ebenen B-B in den Figuren 6 bis 9 jeweils in gestrichelter Linie die Kontur des vor der Schnittebene bzw. zwischen Betrachter und Schnittebene liegenden proximalen Endes des zweiten Maulteils 26 gezeigt. Ferner ist abweichend von einer reinen Schnittdarstellung in den Darstellungen der Schnitte entlang der Ebenen A-A der Figuren 6 und 7 jeweils in gestrichelten Linien die vor der Schnittebene liegende bzw. zwischen dem Betrachter und der Schnittebene liegende Verriegelungseinrichtung 48 angedeutet.

Da das erste Maulteil 25 in den Darstellungen der Schnitte entlang der Ebene B-B jeweils weitgehend verdeckt ist, sind abweichend von einer reinen Schnittdarstellung jeweils auch in gestrichelter Linie der vor der Schnittebene liegende Abschnitt des zweiten Maulteils 26 und in punktierter Linie der vor der Schnittebene liegende zweite Holm 233 der Gelenkeinrichtung 23 angedeutet.

Das proximale Ende des zweiten Maulteils 26 weist einen sich senkrecht zu den Schnittebenen der Figuren 6 bis 9 erstreckenden Drehzapfen 265 auf, der spielarm und reibungsarm in eine entsprechende, sich senkrecht zur Längsachse 29 des Werkzeugs 20 erstreckende Nut bzw. Kerbe am Rand des vor der dargestellten Schnittebene liegenden Holms eingreift. Damit definiert der Drehzapfen 265 am zweiten Maulteil 26 bzw. die Symmetrieachse des Drehzapfens 265 die Schwenkachse 269 des zweiten Maulteils 26.

Der proximale Bereich des zweiten Maulteils 26 weist ferner einen vom Drehzapfen 265 beabstandeten Schlitz 267 auf. Das distale Ende der Übertragungsstange 40 ist als Platte 46 mit einem Steuerzapfen 466 ausgebildet. Der Steuerzapfen 466 greift in den Schlitz 267 am zweiten Maulteil 26 ein. Im Vergleich der Figuren 6 und 7 einerseits sowie 8 und 9 andererseits ist erkennbar, dass eine Linearbewegung der Übertragungsstange 40 zwischen der in den Figuren 6 und 7 dargestellten distalen Position und der in Figur 9 dargestellten proximalen Position aufgrund des Eingriffes des Steuerzapfens 466 in den Mitnahmeschlitz 267 am zweiten Maulteil 26 eine Schwenkbewegung des zweiten Maulteils 26 bewirkt.

Das in den Figuren 6 bis 9 weitgehend verdeckte erste Maulteil 25 ist bezüglich der Längsachse 29 des Werkzeugs 20 symmetrisch zum zweiten Maulteil 26. Anders ausgedrückt geht das erste Maulteil 25 durch eine Drehung um die Längsachse um 180 Grad aus dem zweiten Maulteil hervor und umgekehrt. Entsprechendes gilt für die beiden Holme 231, 233, von denen in den Figuren 6 bis 9 der zweite Holm 233 in punktierter Linie angedeutet ist.

Insbesondere weist das erste Maulteil 25 einen Drehzapfen auf, der spielarm und reibungsarm in eine Kerbe am ersten Holm 231 eingreift. Drehzapfen und Kerbe bilden eine in den Figuren 6 bis 9 angedeutete Drehachse 259 des ersten Bauteils 25, die senkrecht zur Schnittebene der Figuren 6 bis 9 ist. Ferner weisen das proximale Ende des ersten Maulteils 25 einen Schlitz und die Platte 46 am distalen Ende der Übertragungsstange 40 einen Steuerzapfen 465, der hinter der dargestellten Schnittebene liegt und in den Schlitz am proximalen Ende des ersten Maulteils 25 eingreift. Der Steuerzapfen 465 und der in den Figuren 6 bis 9 nicht dargestellte Schlitz im ersten Maulteil 25 bewirken bei einer linearen Bewegung der Übertragungsstange 40 Schwenkbewegung des ersten Maulteils 25 symmetrisch zur Schwenkbewegung des zweiten Maulteils 26.

Zur Führung der Platte 46 am distalen Ende der Übertragungsstange 40 ist ein Führungsstift 234 vorgesehen, der sich in Richtung senkrecht zu den Schnittebenen der Figuren 6 bis 9 zwischen den beiden Holmen 231, 233 der Gelenkeinrichtung 23 erstreckt. Der Führungsstift 234 greift in einen sich in axialer Richtung erstreckenden geraden Führungsschlitz 464 in der Platte 46 am distalen Ende der Übertragungsstange 40 ein. Ferner greift der Führungsstift 234 in bogenförmige Schlitze 254, 264 in den Maulteilen 25, 26 ein. Durch den Eingriff des Führungsstifts 234 in die bogenförmigen Führungsschlitze 254, 264 an den Maulteilen 25, 26 ist gewährleistet, dass die Drehzapfen 265 in den entsprechenden Schlitzen bzw. Kerben in den Holmen 231 der Gelenkeinrichtung 23 verbleiben.

Bei den Darstellungen der Figuren 6 und 7 befinden sich die Maulteile 25, 26 in ihren überoffenen Positionen 253, 263 und die Verriegelungseinrichtung 48 in ihrer Montageposition 483. Das Verbindungsbauteil 27 ist in den Figuren 6 und 7 in hinsichtlich der Rotation um die Längsachse 29 des Werkzeugs 20 unterschiedlichen Orientierungen gezeigt. Bei der in Figur 6 gezeigten Orientierung des Verbindungsbauteils 27 befinden sich die axialen Schlitze 278, die Zapfen 486 und die Köpfe 487 außerhalb der Schnittebene B-B, und Knaggen 226 am proximalen Rand des Verbindungsbauteils 27 in der Schnittebene B-B. Bei dieser Orientierung des Verbindungsbauteils 27 relativ zum distalen Ende 31 des Schafts 30 können die Knaggen 226, wie in Figur 6 angedeutet, in die in axialer Richtung sich erstreckenden Abschnitte der L-förmigen Schlitze 306 eingeführt werden. Die Knaggen 226 am proximalen Rand des Verbindungsbauteils 27 des Werkzeugs 20 und die L-förmigen Schlitze 306 am distalen Ende des Schafts 30 bilden somit Kupplungseinrichtungen oder Bestandteile von Kupplungseinrichtungen zur lösbaren mechanischen Kopplung des Verbindungsbauteils 27 mit dem distalen Ende 31 des Schafts 30.

Wenn die Knaggen 226 ganz in die in axialer Richtung sich erstreckenden Abschnitte der L-förmigen Schlitze 306 in der Bajonetthülse 304 eingeführt sind, kann das Verbindungsbauteil 27 relativ zum Schaft 30 im Uhrzeigersinn (bezogen auf die Darstellungen der Schnitte entlang der Ebenen A-A in den Figuren 6 bis 9) gedreht werden. Dabei werden die Knaggen 226 in den in umfänglicher Richtung sich erstreckenden Abschnitten der L-förmigen Schlitze 306 in der Bajonetthülse 304 verschoben. Nach der Drehung des Verbindungsbauteils 27 relativ zum Schaft 30 ist zwischen dem Werkzeug 20 und dem Schaft 30 eine bezogen auf axiale Zugkräfte formschlüssige Verbindung hergestellt.

Solange die Verriegelungseinrichtung 48 sich in der in den Figuren 6 und 7 dargestellten Montageposition befindet, kann die formschlüssige Verbindung zwischen Werkzeug 20 und Schaft 30 jedoch durch eine entgegengesetzte Drehung bzw. Rotation des Verbindungsbauteils 27 (gegen den Uhrzeigersinn) relativ zum Schaft 30 wieder gelöst werden.

Wenn die Verriegelungseinrichtung 48 sich in den in den Figuren 8 und 9 gezeigten Arbeitspositionen 482, 481 befindet, greifen die radial außen liegenden Enden der Zapfen 486 und die an den radial außen liegenden Enden der Zapfen 486 angeordneten Köpfe 487 in die axialen Abschnitte der L-förmigen Schlitze 306 in der Bajonetthülse 304 ein. Das Verbindungsbauteil 27 kann nicht relativ zum Schaft 30 gedreht werden, und die mechanische Verbindung bzw. Kopplung zwischen Werkzeug 20 und Schaft 30 ist verriegelt.

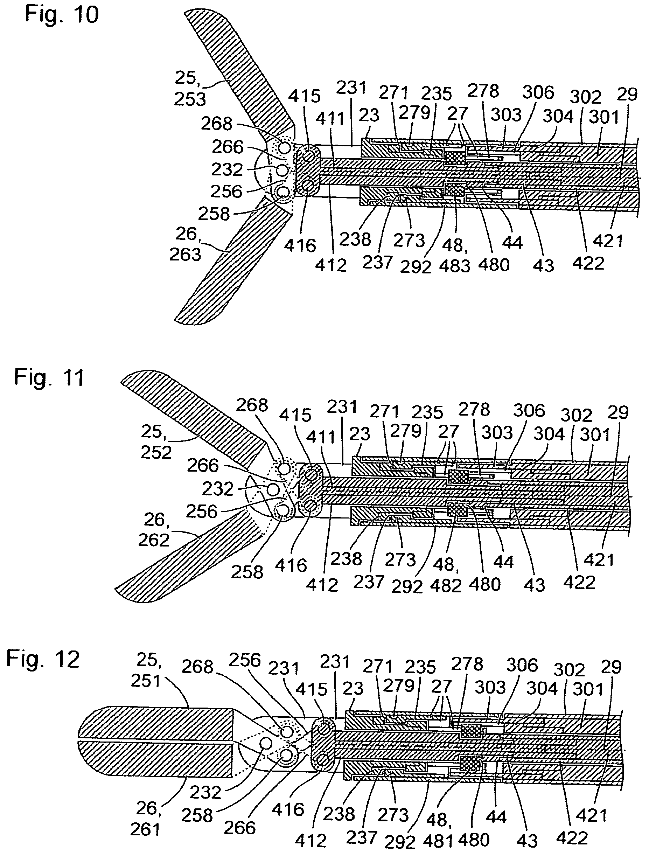

Die Figuren 10 bis 12 zeigen schematische Schnittdarstellungen eines weiteren Ausführungsbeispiels des oben anhand der Figuren 1 und 2 dargestellten Werkzeugs 20. Die Schnittebenen der Figuren 10 bis 12 sind parallel zu den Zeichenebenen der Figuren 1 und 2, entsprechen den Schnittebenen der Figuren 3 bis 5 und den Schnittebenen B-B der Figuren 6 bis 9 und enthalten die in den Figuren 1 und 2 angedeutete Längsachse 29 des Werkzeugs 20. Zusätzlich zum Werkzeug 20 ist in jeder der Figuren 10 bis 12 das distale Ende 31 des Schafts 30 gezeigt. In Figur 10 sind die Maulteile 25, 26 in ihren überoffenen Positionen 253, 263 dargestellt, bei denen die mechanische Verbindung zwischen Werkzeug 20 und Schaft 30 entriegelt ist, d.h. hergestellt oder gelöst werden kann. In Figur 11 sind die Maulteile 25, 26 in ihren offenen Positionen 252, 262 dargestellt, die Verbindung zwischen Werkzeug 20 und Schaft 30 ist verriegelt. In Figur 12 sind die Maulteile 25, 26 in ihren geschlossenen Positionen 251, 261 dargestellt, die Verbindung zwischen Werkzeug 20 und Schaft 30 ist ebenfalls verriegelt.

Das Ausführungsbeispiel der Figuren 10 bis 12 ähnelt in einigen Merkmalen dem Ausführungsbeispiel der Figuren 3 bis 5 und in geringerem Maße auch dem Ausführungsbeispiel der Figuren 6 bis 9. Merkmale des Ausführungsbeispiels der Figuren 10 bis 12, die Merkmalen des Ausführungsbeispiels der Figuren 3 bis 5 ähneln, sind nicht nochmals beschrieben. Wie nachfolgend beschrieben, unterscheidet sich das Ausführungsbeispiel der Figuren 10 bis 12 von dem Ausführungsbeispiel der Figuren 3 bis 5 insbesondere bei der Übertragungsstange 40, bei der Verriegelungseinrichtung 48 und bei der mechanischen Verbindung des proximalen Bereichs der Gelenkeinrichtung 23mit dem Verbindungsbauteil 27.

Die Gelenkeinrichtung 23 weist in ihrem proximalen Bereich einen Querschnitt mit kreisförmiger oder im Wesentlichen kreisförmiger Kontur auf, der sich von distal nach proximal in mehreren Stufen verringert. Ein kreisringförmiges Kontaktbauteil 238 ist angrenzend an eine kreisringförmige, ebene und nach proximal orientierte Stirnfläche der Gelenkeinrichtung 23, die durch eine dieser Stufen gebildet ist, angeordnet. Das Kontaktbauteil 238 weist ein Metall oder ein anderes elektrisch leitfähiges und insbesondere lötbares oder schweißbares Material auf.

Ein Ring 235 ist mechanisch starr mit dem proximalen Ende der Gelenkeinrichtung 23 verbunden. Beispielsweise weisen die Gelenkeinrichtung 23 an ihrem proximalen Ende ein Außengewinde und der Ring 235 ein entsprechendes Innengewinde auf. Alternativ oder zusätzlich kann der Ring 235 mit der Gelenkeinrichtung 23 durch Klebstoff, durch Schweißen oder Löten verbunden sein.

Zwischen dem Kontaktbauteil 238 und dem Ring 235 weist die Gelenkeinrichtung 23 eine ringförmige Nut 237 auf, deren kreisringförmige Flanken durch die proximale Stirnseite des Kontaktbauteils 238 und die distale Stirnseite des Rings 235 gebildet werden. In diese Nut 237 greift ein nach radial innen vorstehender Kragen 273 eines Verbindungsbauteils 27 ein, das weitgehend dem Verbindungsbauteil des Ausführungsbeispiels der Figuren 3 bis 5 ähnelt. Zur Montage werden zunächst der Kragen 273 des Verbindungsbauteils 27 an das Kontaktbauteil 238 herangeführt und dann der Ring 235 an der Gelenkeinrichtung 23 befestigt.

Der Kragen 273 und die Nut 237 weisen einander entsprechende Oberflächen auf. Durch den Eingriff des Kragens 273 in die Nut 237 ist das Verbindungsbauteil 27 spielarm und reibungsarm formschlüssig an der Gelenkeinrichtung 23 gehalten. Das Verbindungsbauteil 27 ist relativ zu der Gelenkeinrichtung 23 nur um die Längsachse 29 des Werkzeugs 20 rotierbar, jedoch - bis auf erwünschtes oder unvermeidbares Spiel - weder parallel zur Längsachse 29 bewegbar noch um Achsen senkrecht zur Längsachse 29 kippbar.

Das Werkzeug 20 weist im Bereich des Verbindungsbauteils 27 eine im Wesentlichen kreiszylindermantelförmige Isolierhülle 292 auf, die in Richtung parallel zur Längsachse 29 des Werkzeugs 20 nach distal und insbesondere auch nach proximal gegenüber dem Verbindungsbauteil 27 übersteht. Insbesondere ist der distale Rand der Isolierhülle 292 distal des Kontaktbauteils 238 angeordnet.

Die innere Oberfläche der Isolierhülle 292 ist zumindest in einem Teil des Bereichs, in dem sie an der äußeren Oberfläche des Verbindungsbauteils 27 anliegt, an der äußeren Oberfläche des Verbindungsbauteils 27 durch eine Klebung oder auf andere Weise stoffschlüssig und/oder formschlüssig und/oder kraftschlüssig gefügt. Insbesondere weist die äußere Oberfläche des Verbindungsbauteils 27 eine Längsriffelung oder eine andere Strukturierung auf, die eine Übertragung eines Drehmoments von der Isolierhülle 292 auf das Verbindungsbauteil 27 unterstützt.

Das Verbindungsbauteil 27 weist im Bereich des Kragens 273 an seiner äußeren Oberfläche eine ringförmige Nut 279 auf, die bei dem hier dargestellten Beispiel einen halbkreisförmigen Querschnitt aufweist. Die Nut 279 ist insbesondere ausgebildet, um beim Aufschieben der Isolierhülle 292 auf das Verbindungsbauteil 27 parallel zur Längsachse 29 des Werkzeugs 20 von proximal nach distal überschüssigen Klebstoff aufzunehmen. Insbesondere wirkt die Kante am distalen Rand der Nut 279 als Abstreifkante für Klebstoff, der sich an der Innenseite der Isolierhülle 292 befindet. Damit kann verhindert werden, dass dieser überschüssige Klebstoff distal des Verbindungsbauteils 27 eine unerwünschte stoffschlüssige Verbindung der Isolierhülle 292 mit dem Kontaktbauteil 238 oder mit der Gelenkeinrichtung 23 bildet.

Die Übertragungsstange 40 unterscheidet sich von der Übertragungsstange des Ausführungsbeispiels der Figuren 3 bis 5 insbesondere durch eine Isolierhülle 412. Die Isolierhülle 412 erstreckt sich annähernd über die gesamte Länge des distalen Abschnitts 411 der Übertragungsstange 40 und umschließt den distalen Abschnitt 411 der Übertragungsstange 40 mantelförmig, insbesondere im Wesentlichen kreiszylindermantelförmig. Die äußere Kontur des Querschnitts der Isolierhülle 412 ist kreisförmig, wobei der Radius sich an einer Stelle stufenförmig ändert. Nahe dem proximalen Ende der Isolierhülle 412 ist ein Ring 44 angeordnet. Der Ring 44 weist insbesondere ein elektrisch isolierendes Material auf und umschließt die Isolierhülle 412. Der Ring 44 ist insbesondere stoffschlüssig und/oder kraft- bzw. reibschlüssig mit der Isolierhülle 412 verbunden.

Ein Isoliermantel 422 umschließt den proximalen Abschnitt 421 der Übertragungsstange 40 mantelförmig, insbesondere kreiszylindermantelformig. Nahe seinem distalen Ende überlappt der Isoliermantel 422 teilweise mit dem Ring 44. Die Isolierhülle 412, der Ring 44 und der Isoliermantel 422 bilden somit eine vollständige elektrische Isolation der Übertragungsstange in dem Bereich, in dem die Übertragungsstange 40 innerhalb des Werkzeugs 20 und/oder innerhalb des Schafts 30 angeordnet ist. Lediglich im Bereich der Gelenke 415, 416 am distalen Ende der Übertragungsstange 40 ist die Übertragungsstange 40 nicht elektrisch isoliert. Die Leitfähigkeit der Materialien des distale Abschnitts 411 und des proximalen Abschnitts 421 der Übertragungsstange 40 sowie der Verbindungshülse 43 ermöglicht eine Zuführung eines elektrischen Potentials zum distalen Ende der Übertragungsstange 40.

Eine Verriegelungseinrichtung 48 umfasst einen ringförmigen Abschnitt 480. Der ringförmige Abschnitt 480 der Verriegelungseinrichtung 48 ist in einer umlaufenden Nut an der Übertragungsstange 40 zwischen der stufenförmigen Querschnittsänderung der Isolierhülle 412 und dem Ring 44 angeordnet. Die distale kreisringförmige Stirnfläche des ringförmigen Abschnitts 480 liegt an einer nach proximal orientierten kreisringförmigen Fläche an, die durch die stufenförmige Querschnittsänderung der Isolierhülle 412 gebildet ist. Die proximale kreisringförmige Stirnfläche des ringförmigen Abschnitts 480 liegt an einer nach distal orientierten kreisringförmigen Stirnfläche des Rings 44 an. Dadurch ist der ringförmige Abschnitt 480 der Verriegelungseinrichtung 48 in der umlaufenden Nut spielarm und reibungsarm geführt. Somit ist die Verriegelungseinrichtung 48 relativ zur Übertragungsstange 40 um die Längsachse 29 des Werkzeugs 20 rotierbar, aber nicht in Richtung parallel zur Längsachse 29 des Werkzeugs 20 verschiebbar.

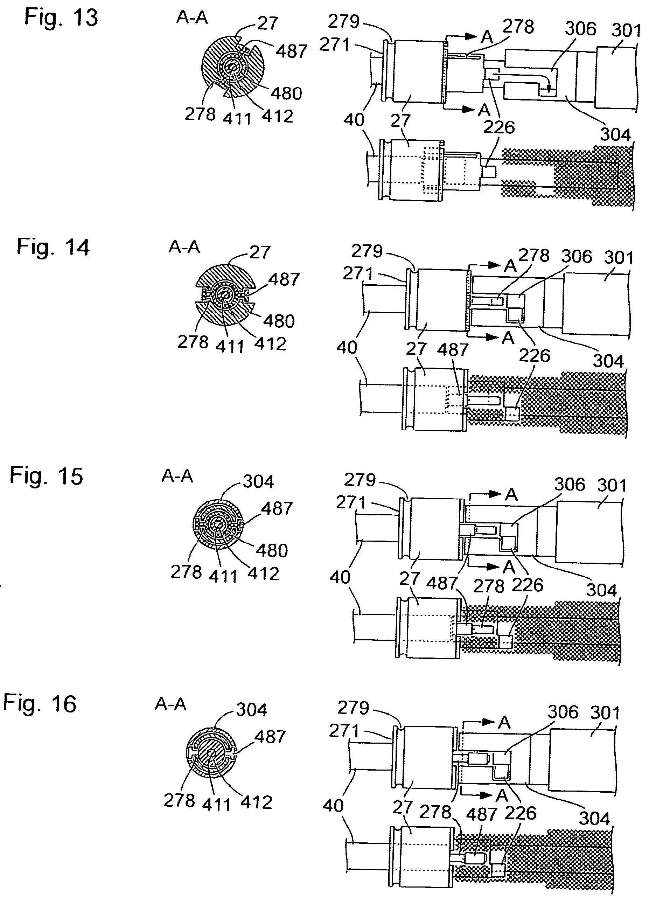

Die Verriegelungseinrichtung 48 unterscheidet sich von den Verriegelungseinrichtungen der Ausführungsbeispiele der Figuren 3 bis 5 und 6 bis 9 insbesondere durch einen einstückigen Aufbau. Anstelle von ringförmigen Köpfen, die an radial äußere Enden von Zapfen gefügt sind weist die Verriegelungseinrichtung 48 des Ausführungsbeispiels der Figuren 10 bis 12 T-förmige Köpfe auf, die mit dem ringförmigen Abschnitt 480 der Verriegelungseinrichtung 48 einstückig hergestellt sind. Zwei Köpfe liegen in der Schnittebene der Figuren 10 bis 12. Wie unten anhand der Figuren 13 bis 16 beschrieben wird, weist jeder Kopf in einer Schnittebene senkrecht zur Längsachse 29 des Werkzeugs 20 einen T-förmigen Querschnitt auf.

Nachfolgend wird mit Bezug auf die Figuren 13 bis 16 eine verriegelnde Wirkung der Verriegelungseinrichtung 48 auf eine mechanische Verbindung bzw. Kopplung des Werkzeugs 20 mit dem distalen Ende 31 eines Schafts 30 (vgl. Figuren 3 bis 5, 6 bis 9 und 10 bis 12) dargestellt. Die mechanische Verbindung bzw. Kopplung zwischen dem Werkzeug 20 und dem Schaft 30 erfolgt mittels des L-förmigen Schlitzes 306 im distalen Bereich der Bajonetthülse 304 am distalen Ende des Schafts 30 und der Knagge 226 am proximalen Ende des Verbindungsbauteils 27 des Werkzeugs 20. In den Figuren 13 bis 16 sind deshalb nur das Verbindungsbauteil 27, das distale Ende des Schafts 30 mit der Bajonetthülse 304 und die Übertragungsstange 40 mit der Verriegelungseinrichtung 48 dargestellt.

In jeder der Figuren 13 bis 16 sind links ein Schnitt entlang einer Ebene A-A senkrecht zur Längsachse 29 des Werkzeugs 20 (vgl. Figuren 3 bis 5, 6 bis 9 und 10 bis 12) und rechts jeweils zwei Draufsichten auf das Verbindungsbauteil 27, das distale Ende des Schafts 30 mit der Bajonetthülse 304 und die Übertragungsstange 40 gezeigt. Die Schnittebenen A-A der Figuren 13 bis 16 unterscheiden sich teilweise durch einen leichten parallelen Versatz. Die Zeichenebenen der Darstellungen in den Figuren 13 bis 16 rechts sind jeweils senkrecht zur Schnittebene A-A, parallel zur Längsachse 29 des Werkzeugs 20 (vgl. Figuren 3 bis 5, 6 bis 9 und 10 bis 12) und senkrecht zu den Schnitt- und Zeichenebenen der Figuren 3 bis 12. In den Figuren 13 bis 16 rechts ist jeweils die Lage der Schnittebene A-A angedeutet.

Die Draufsichten auf das Verbindungsbauteil 27, das distale Ende des Schafts 30 und die Übertragungsstange 40 sind in den Figuren 13 bis 16 rechts jeweils zweimal übereinander gezeigt. Jeweils oben sind die Konturen der Bajonetthülse 304 am distalen Ende des Schafts 30 in durchgezogener Linie dargestellt, die Übertragungsstange 40 ist, soweit sie im Inneren des Verbindungsbauteils 27 oder des Schafts 30 verläuft, nur in gestrichelten Linien dargestellt. Jeweils unten ist die Übertragungsstange 40 - soweit sie nicht im Inneren des Verbindungsbauteils 27 angeordnet ist - in durchgezogenen Linien dargestellt, und der Schaft 30 mit der Bajonetthülse 304 ist lediglich in konturloser Schraffur angedeutet.

In den Figuren 13 bis 16 rechts ist die L-förmige Gestalt jeweils eines Schlitzes 306 im distalen Bereich der Bajonetthülse 304 erkennbar. Die Bajonetthülse 304 umfasst insbesondere zwei L-förmige Schlitze 306, die, bezogen auf die Längsachse 29 des Werkzeugs 20 (vgl. Figuren 3 bis 5, 6 bis 9 und 10 bis 12) um 180° gegeneinander versetzt sind. Jeder L-förmige Schlitz in der Bajonetthülse 304 umfasst einen axialen Abschnitt und einen umfänglichen Abschnitt. Der axiale Abschnitt erstreckt sich in axialer Richtung, der umfängliche Abschnitt erstreckt sich in umfänglicher Richtung.

In den Figuren 13 bis 16 ist ferner der bereits genannte, jedoch in den Figuren 3 bis 5, 6 bis 9 und 10 bis 12 nur schwer erkennbare axiale Schlitz 278 am Verbindungsbauteil 27 erkennbar, der sich in axialer Richtung erstreckt. In den Schnitten entlang den Ebenen A-A ist jeweils erkennbar, dass jeder Kopf 487 einen T-förmigen Querschnitt aufweist und in radialer Richtung durch einen der axialen Schlitze 278 hindurch ragt.

Bei den in den Figuren 13 und 14 dargestellten Situationen befindet sich die Verriegelungseinrichtung 48 in der auch in den Figur 3, 6 und 10 gezeigten Montageposition 483. Der Riegel 484 ist weitgehend innerhalb des Verbindungsbauteils 27 verborgen. Die Knagge 226 kann, wie in Figur 13 durch einen Pfeil angedeutet, durch eine axiale Bewegung und eine anschließende Drehbewegung des Verbindungsbauteils 27 relativ zum Schaft 30 durch den axialen Abschnitt des L-förmigen Schlitzes 306 in den umfänglichen Abschnitt des L-förmigen Schlitzes 306 in der Bajonetthülse eingebracht werden.

Bei den in den Figuren 14, 15 und 16 dargestellten Situationen liegt die Knagge 226 im umfänglichen Abschnitt des L-förmigen Schlitzes 306 in der Bajonetthülse 304 am distalen Ende des Schafts 30. Wenn die Knagge 226 im umfänglichen Abschnitt des L-förmigen Schlitzes 306 liegt, ist durch Formschluss zwischen Knagge 226 und umfänglichem Abschnitt des L-förmigen Schlitzes 306 hinsichtlich Zugkräften eine mechanische Verbindung zwischen dem Verbindungsbauteil 27 und der Bajonetthülse 304 bzw. dem distalen Ende des Schafts 30 gebildet. Damit ist auch eine formschlüssige Verbindung zwischen dem gesamten Werkzeug 20 und dem Schaft 30 gebildet, die Zugkräfte aufnimmt. Somit bilden die Knaggen 226 am proximalen Ende des Verbindungsbauteils 27 des Werkzeugs 20 und die L-förmigen Schlitze 306 in der Bajonetthülse 304 am distalen Ende des Schafts 30 Kupplungseinrichtungen oder sind Bestandteile von Kupplungseinrichtungen zur lösbaren mechanischen Kopplung des Verbindungsbauteils 27 mit dem distalen Ende des Schafts 30.

Solange, wie in den Figuren 13 und 14 gezeigt, die Verriegelungseinrichtung 48 sich in der auch in den Figuren 3, 6 und 10 gezeigten Montageposition 483 befindet, kann die Knagge 226 durch eine Drehbewegung und eine anschließende axiale Bewegung des Verbindungsbauteils 27 relativ zum Schaft 30 wieder aus dem L-förmigen Schlitz 306 in der Bajonetthülse 304 am distalen Ende des Schafts 30 entfernt werden.

Bei der in Figur 15 dargestellten Situation befindet sich die Verriegelungseinrichtung 48 in der auch in den Figuren 4, 8 und 11 gezeigten zweiten Arbeitsposition 482. Bei der in Figur 16 gezeigten Situation befindet sich die Verriegelungseinrichtung 48 in der auch in den Figuren 6, 9 und 12 gezeigten ersten Arbeitsposition 481. In allen Positionen 481, 482, 483 greift jeder der T-förmigen Köpfe 487 der Verriegelungseinrichtung 48 jeweils in radialer Richtung durch einen Schlitz 278 am proximalen Ende des Verbindungsbauteils 27 hindurch. Sowohl in der ersten Arbeitsposition 481 als auch in der zweiten Arbeitsposition 482 der Verriegelungseinrichtung 48 greift jeder der T-förmigen Köpfe 487 der Verriegelungseinrichtung 48 jeweils ferner in den axialen Abschnitt eines L-förmigen Schlitzes 306 in der Bajonetthülse ein. Somit blockieren die Verriegelungseinrichtung 48 und insbesondere ihre T-förmigen Köpfe 487 in den Arbeitspositionen 481, 482 eine relative Rotation von Verbindungsbauteil 27 und Bajonetthülse 304. Damit hält bzw. blockiert die Verriegelungseinrichtung 48 die Knagge 226 im umfänglichen Abschnitt des L-förmigen Schlitzes 306 in der Bajonetthülse 304. Somit verriegelt die Verriegelungseinrichtung 48, in den in den Figuren 4, 5, 8, 9, 11 und 12 gezeigten Arbeitspositionen 481, 482 die mechanische Verbindung bzw. Kopplung des Werkzeugs 20 mit dem distalen Ende des Schafts 30.

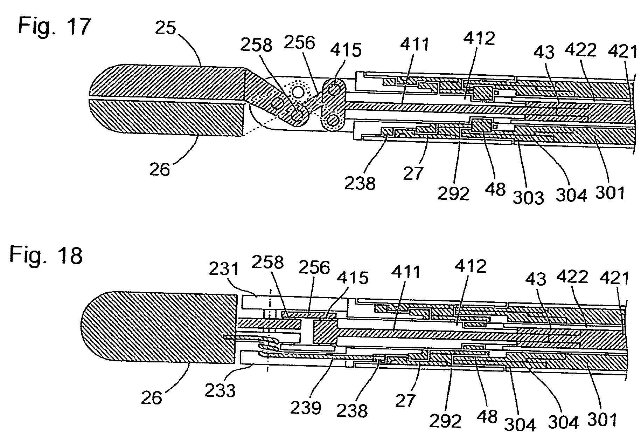

Der anhand der Figuren 10 bis 12 gezeigte Aufbau des Werkzeugs 20 eignet sich für ein bipolares mikroinvasiv-chirurgisches Instrument, bei dem zwischen den Maulteilen 25, 26 eine hochfrequente Wechselspannung angelegt wird, um durch die Maulteile 25, 26 gegriffenes oder zerschnittenes Gewebe durch Stromfluss zu veröden, insbesondere Eiweiß zu koagulieren. Nachfolgend wird die Kontaktierung der Maulteile 25, 26 bzw. die Zuführung elektrischen Stroms und elektrischer Leistung zu den Maulteilen 25, 26 anhand der Figuren 17 und 18 beschrieben.

In den Figuren 17 und 18 ist das Werkzeug 20 mit dem distalen Ende 31 des Schafts 30 in schematischen Schnittdarstellungen gezeigt. Die Schnittebene der Figur 17 entspricht den Schnittebenen der Figuren 3 bis 12. Die Schnittebene der Figur 18 ist senkrecht zu den Schnittebenen der Figuren 3 bis 12 und 17, parallel zu den Zeichenebenen der Darstellungen in den Figuren 13 bis 16 rechts und parallel zur Längsachse 29 des Werkzeugs 20.

In den Figuren 17 und 18 sind alle elektrisch leitfähigen Elemente, die mit dem ersten Maulteil 25 elektrisch leitfähig verbunden sind, mit einer Schraffur von links unten nach rechts oben versehen. Alle Elemente, die mit dem zweiten Maulteil 26 elektrisch leitfähig verbunden sind, sind mit einer Schraffur von rechts unten nach links oben versehen. Elemente, die elektrisch isolierende Materialien aufweisen, sind ebenso wie Hohlräume nicht schraffiert und nur teilweise mit Bezugszeichen versehen. Um die elektrisch leitfähige Verbindung zu den Maulteilen darzustellen, weichen die Figuren 17 und 18 im Bereich links der Bildmitte bzw. distal der Gelenke 415, 416 am distalen Ende der Übertragungsstange 40 von einer Schnittdarstellung ab. In diesem distalen Bereich entspricht die Figur 18 eher einer Draufsicht.

Das erste Maulteil 25 ist über den proximalen Abschnitt 421 der Übertragungsstange 40, die Verbindungshülse 43, den distalen Abschnitt 411 der Übertragungsstange 40, das Gelenk 415, das erste Pleuel 256 und das Gelenk 258 am ersten Maulteil 25 elektrisch kontaktiert. Das zweite Maulteil 26 ist über das Metallrohr 301 des Schafts 30, die Bajonetthülse 304, die in keiner der beiden Schnittebenen der Figuren 10 und 11 liegende Knagge 226 (vgl. Figuren 6 und 13 bis 16), die Verriegelungseinrichtung 48, das Verbindungsbauteil 27, das Kontaktbauteil 238 und ein nur in Figur 18 dargestelltes elastisches elektrisch leitfähiges Bauteil 239 kontaktiert.

Das elastische elektrisch leitfähige Bauteil 239 wird insbesondere durch einen metallischen Draht gebildet, der einen helikalen Abschnitt an der Welle 232 aufweist. Das proximale Ende des elastischen elektrisch leitfähigen Bauteils 239 ist mit dem Kontaktbauteil 238 verbunden, beispielsweise gesteckt, geklemmt, gelötet oder verschweißt. Insbesondere ist das proximale Ende des elastischen elektrisch leitfähigen Bauteils 239 abweichend von der Darstellung in Figur 11 in das Lumen eines mit dem Kontaktbauteil 238 verschweißten oder verlöteten Rohres gesteckt und optional mit dem Rohr verschweißt oder verlötet. Eine leichte Krümmung des proximalen Endes des elastischen elektrisch leitfähigen Bauteils 239 kann eine elastische Klemmung des proximalen Endes des elastischen elektrisch leitfähigen Bauteils 239 in dem Rohr und damit einen zuverlässigen Kontakt bewirken, wenn das elastische elektrisch leitfähige Bauteil 239 nicht mit dem Rohr oder dem Kontaktbauteil 238 verschweißt oder verlötet ist. Das distale Ende des elastischen elektrisch leitfähigen Bauteils 239 ist elektrisch leitfähig mit dem zweiten Maulteil 26 verbunden, beispielsweise geklemmt, gesteckt, verlötet oder verschweißt.

Der proximale Abschnitt des elastischen elektrisch leitfähigen Bauteils 239 ist insbesondere in einem der Holme 231, 233 der Gelenkeinrichtung 23 angeordnet. Ein distaler Abschnitt des elastischen elektrisch leitfähigen Bauteils 239 ist insbesondere in einem elektrisch isolierenden Abschnitt des zweiten Maulteils 26 angeordnet, der das Lager an der Welle 232 und das Gelenk 268 zum zweiten Pleuel 266 umfasst.

Die kreisringförmige proximale Stirnfläche des Kontaktbauteils 238 liegt an der kreisringförmigen distalen Stirnfläche des Verbindungsbauteils 27 an. Der mechanische Kontakt beider Stirnflächen bildet gleichzeitig einen elektrisch leitfähigen Kontakt. Die proximale Stirnfläche des Kontaktbauteils 238 und die distale Stirnfläche des Verbindungsbauteils 27 sind somit korrespondierende Kontaktflächen. Der mechanische und elektrische Kontakt besteht unabhängig von der rotatorischen Position des Werkzeugs 20 relativ zu dem Verbindungsbauteil 27 und dem mit dem Verbindungsbauteil 27 mechanisch und elektrisch gekoppelten Bajonetthülse 304.

Wenn die Maulteile 25, 26 durch eine auf die Übertragungsstange 40 wirkende Zugkraft nach proximal in Richtung auf die geschlossene Positionen 251, 261 bewegt werden, werden die proximale Stirnfläche des Kontaktbauteils 238 und die distale Stirnfläche des Verbindungsbauteils 27 zusammengedrückt. Deshalb besteht der mechanische und elektrische Kontakt zwischen Kontaktbauteil 238 und Verbindungsbauteil 27 selbst im Fall eines mechanischen Spiels des Kragens 273 in der Nut 237 zumindest dann, wenn die Maulteile 25, 26 gegen einen mechanischen Widerstand geschlossen werden.

Bei den oben dargestellten Ausführungsformen ist die Übertragungsstange 40 sowohl zur Übertragung einer translatorischen Bewegung in einer Richtung parallel zum Schaft 30 bzw. zu dessen Längsachse und einer entsprechenden Zug- und/oder Schubkraft als auch zur Übertragung einer rotatorischen Bewegung und eines entsprechenden Drehmoments zum Werkzeug 20 ausgebildet. Abweichend von der Darstellung anhand der Figuren kann das Werkzeug 20 am distalen Ende 31 des Schafts 30 lediglich um die Längsachse des distalen Endes 31 des Schafts 30 oder um eine andere Achse rotierbar sein. Beispielsweise umfasst das Werkzeug 20 einen fingerförmigen oder anderen Manipulator oder eine Elektrode in Haken- oder Schlaufenform oder in anderer Gestalt. In diesem Fall ist eine Ausbildung der Übertragungsstange 40 für die Übertragung einer translatorischen Bewegung in einer Richtung parallel zum Schaft 30 und für die Übertragung einer entsprechenden Kraft nur insoweit erforderlich, als sie zum Bewegen der Verriegelungseinrichtung 48 erforderlich ist.

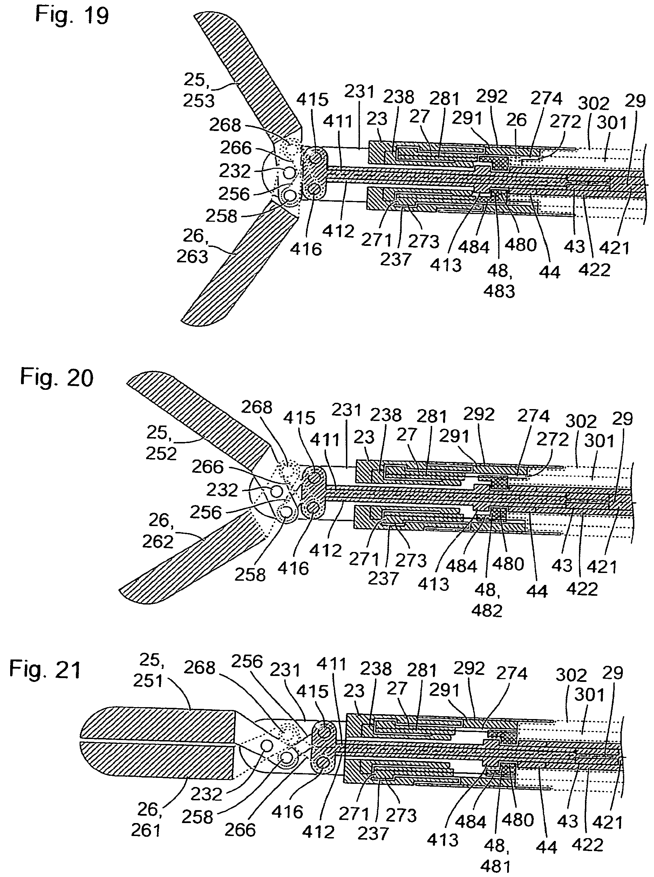

Die Figuren 19 bis 21 zeigen schematische Schnittdarstellungen eines weiteren Ausführungsbeispiels des oben anhand der Figuren 1 und 2 dargestellten Werkzeugs 20. Die Schnittebenen der Figuren 19 bis 21 sind parallel zu den Zeichenebenen der Figuren 1 und 2, entsprechen unter anderem den Schnittebenen der Figuren 3 bis 5 und 10 bis 12 und enthalten die in den Figuren 1 und 2 angedeutete Längsachse 29 des Werkzeugs 20. Zusätzlich zum Werkzeug 20 ist in jeder der Figuren 19 bis 21 in gestrichelten Linien das distale Ende 31 des Schafts 30 gezeigt. In Figur 19 sind die Maulteile 25, 26 in ihren überoffenen Positionen 253, 263 dargestellt, bei denen die mechanische Verbindung zwischen Werkzeug 20 und Schaft 30 entriegelt ist, d.h. hergestellt oder gelöst werden kann. In Figur 20 sind die Maulteile 25, 26 in ihren offenen Positionen 252, 262 dargestellt, die Verbindung zwischen Werkzeug 20 und Schaft 30 ist verriegelt. In Figur 21 sind die Maulteile 25, 26 in ihren geschlossenen Positionen 251, 261 dargestellt, die Verbindung zwischen Werkzeug 20 und Schaft 30 ist ebenfalls verriegelt.

Das Werkzeug weist eine Gelenkeinrichtung 23 auf, die in einem distalen Bereich gabelförmig mit zwei Holmen ausgebildet ist. Ein Holm 231 liegt hinter den Schnittebenen der Figuren 19 bis 21 und ist in den Figuren 19 bis 21 erkennbar. Ein zweiter Holm ist bezüglich den Schnittebenen der Figuren 19 bis 21 symmetrisch zu dem in den Figuren 19 bis 21 gezeigten Holm 231 ausgebildet und angeordnet. Der zweite Holm liegt vor der Schnittebene der Figuren 19 bis 21 und ist deshalb in den Figuren 19 bis 21 nicht dargestellt.

Zwischen den beiden Holmen ist eine Welle 232 angeordnet, deren Achse senkrecht zu den Schnitt- und Zeichenebenen der Figuren 19 bis 21 ist. Die Enden der Welle 232 sind in je einem der Holme 231 befestigt. Die Maulteile 25, 26 sind jeweils um eine Schwenkachse senkrecht zu den Schnitt- und Zeichenebenen der Figuren 19 bis 21 drehbar auf der Welle 232 gelagert.

In einem proximalen Bereich ist die Gelenkeinrichtung 23 rotationssymmetrisch oder im Wesentlichen rotationssymmetrisch zur Längsachse 29 des Werkzeugs 20 und im Wesentlichen hülsenförmig. In diesem Bereich weist die Gelenkeinrichtung 23 eine umlaufende bzw. ringförmige Nut 237 auf, die sich nach innen öffnet bzw. von der Innenseite der dort hülsenförmige Gelenkeinrichtung 23 ausgeht.

Das Werkzeug 20 umfasst ferner ein Verbindungsbauteil 27. Das Verbindungsbauteil 27 weist eine hülsenförmige bzw. rohrförmige bzw. kreiszylindermantelförmige Grundgestalt auf, die im Wesentlichen rotationssymmetrisch zur Längsachse 29 des Werkzeugs 20 ist. Das Verbindungsteil weist einige Abweichungen von der Rotationssymmetrie auf, die nachfolgend anhand der Figuren 19 bis 25 dargestellt sind.

Das Verbindungsbauteil 27 weist nahe seinem distalen Rand 271 einen Kragen 273 auf, der in die umlaufende Nut 237 an der Gelenkeinrichtung 23 eingreift. Der Kragen 273 an dem Verbindungsbauteil 27 und die umlaufende Nut 237 an der Gelenkeinrichtung 23 bilden eine formschlüssige und spielarme permanente mechanische Verbindung zwischen der Gelenkeinrichtung 23 und dem Verbindungsbauteil 27. Die durch den Kragen 273 am Verbindungsbauteil und die Nut 237 an der Gelenkeinrichtung 23 gebildete mechanische Verbindung zwischen Verbindungsbauteil 27 und Gelenkeinrichtung 23 erlaubt eine freie Rotation der Gelenkeinrichtung 23 relativ zum Verbindungsbauteil 27 um die Längsachse 29 des Werkzeugs 20. Gleichzeitig unterbindet die durch den Kragen 273 am Verbindungsbauteil und die Nut 237 an der Gelenkeinrichtung 23 gebildete mechanische Verbindung relative axiale Verschiebung parallel zur Längsachse 29 des Werkzeugs 20 und eine relative Kippbewegungen um Achsen senkrecht zur Längsachse 29 des Werkzeugs 20.

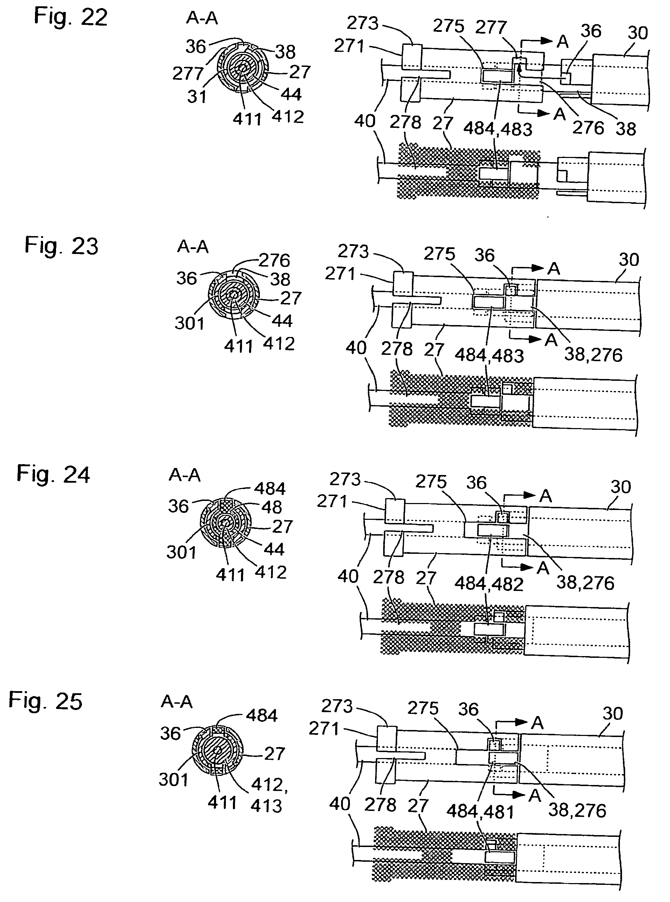

Das Verbindungsbauteil 27 weist ferner von seinem distalen Ende 27 ausgehende radiale Schlitze auf, die in den Figuren 19 bis 21 nicht erkennbar sind und unten mit Bezug auf die Figuren 22 bis 25 beschrieben werden. Ferner weist das Verbindungsbauteil 27 von seinem proximalen Ende 272 ausgehende T-förmige Schlitze 274 auf, die in den Figuren 19 bis 21 teilweise erkennbar sind.

An der inneren Oberfläche des Verbindungsbauteils 27, die von den erwähnten axialen Schlitzen abgesehen im Wesentlichen kreiszylindermantelförmig ist, liegt die äußere Oberfläche einer Stützhülse 281 an. Die Stützhülse 281 ist im Wesentlichen rohrförmig und weist insbesondere eine geringere Länge auf als das Verbindungsbauteil 27.

Im Bereich des distalen Endes des hülsenförmigen Abschnitts der Gelenkeinrichtung 23 ist ein Kontaktbauteil 238 formschlüssig, stoffschlüssig oder kraftschlüssig mit der Gelenkeinrichtung 23 verbunden, das sich radial innerhalb der Stützhülse 281 hülsenförmig nach proximale erstreckt und nahe seinem proximalen Ende mit einer Kontaktfläche an der Innenseite der Stützhülse 281 anliegt.

Ein im Wesentlichen kreiszylindermantelformiger Handhabungsring 291 umschließt einen proximalen Bereich des Verbindungsbauteils 27 und ist mit diesem insbesondere stoffschlüssig und/oder kraftschlüssig und/oder formschlüssig verbunden. Eine im Wesentlichen kreiszylindermantelförmige Isolierhülle 292 umschließt den Handhabungsring 291 und steht gegenüber diesem in beiden axialen Richtungen über. Die Isolierhülle 292 ist stoffschlüssig und/oder kraftschlüssig und/oder formschlüssig mit dem Handhabungsring 291 verbunden.

Das Verbindungsbauteil 27, die Stützhülse 281, der Handhabungsring 291 und die Isolierhülle 292 bilden somit eine starre mechanische Einheit, die von medizinischem Personal an der äußeren Oberfläche der Isolierhülle 292 gefasst und gegenüber der Gelenkeinrichtung 23 gedreht werden kann. Die äußere Oberfläche der Isolierhülle ist insbesondere zur Handhabung durch medizinisches Personal während des mechanischen Koppelns des Werkzeugs 20 mit einem distalen Ende 31 eines Schafts 30 vorgesehen.

Das in den Figuren 19 bis 21 in gestrichelten Linien angedeutete distale Ende eines Schafts 30 (vgl. Figuren 1 und 2) umfasst ein Metallrohr 301 oder ein anderes biegesteifes Rohr aus einem nichtmetallischen Material, das von einem Isoliermantel 302 umschlossen ist. Nahe dem distalen Rand des Metallrohres 301 sind mehrere Knaggen 36 angeordnet. Die Knaggen 36 sind hinsichtlich ihrer Anordnung und ihrer Gestalt so ausgebildet, dass sie in der Art einer Bajonettkupplung in die in den Figuren 19 bis 21 nur teilweise erkennbaren T-förmigen Schlitze 274 am Verbindungsbauteil 27 angreifen können, um eine lösbare mechanische Verbindung zwischen dem distalen Ende des Schafts 30 und dem Verbindungsbauteil 27 zu bilden. Die T-förmigen Schlitze 274 am Verbindungsbauteil 27 und die Knaggen 36 am distalen Rand des Metallrohres 301 bilden somit Kupplungseinrichtungen oder sind Bestandteile von Kupplungseinrichtungen zur lösbaren mechanischen Kopplung des Verbindungsbauteils 27 mit dem distalen Ende 31 des Schafts 30.

Die bereits in Figur 2 skizzierte Übertragungstange 40 umfasst in dem in den Figuren 19 bis 21 dargestellten distalen Bereich einen distalen Abschnitt 411. Der distale Abschnitt 411 der Übertragungsstange 40 ist - vom äußersten distalen Ende abgesehen - von einer Isolierhülle 412 mantelförmig umgeben, die in einem mittleren Bereich einen Kragen 413 umfasst. Am äußersten distalen Ende weist der distale Abschnitt 411 der Übertragungsstange 40 zwei Gelenke 415, 416 auf. Ein erstes Pleuel 256 verbindet das erste Gelenk 415 am distalen Ende der Übertragungsstange 40 mit einem von der Welle 232 beabstandeten Gelenk 258 am ersten Maulteil 25. Ein zweites Pleuel 266 verbindet das zweite Gelenk 416 am distalen Ende der Übertragungsstange 40 mit einem von der Welle 232 beabstandeten Gelenk 268 am zweiten Maulteil 26. In der Zusammenschau der Figuren 19 bis 21 ist erkennbar, dass eine lineare Verschiebung der Übertragungsstange 40 parallel zur Längsachse 29 des Werkzeugs 20 vermittels der Pleuel 256, 266 ein Schwenken der Maulteile 25, 26 um die durch die Welle 232 gebildeten Gelenke bewirkt.

Die Übertragungsstange 40 umfasst ferner einen proximalen Abschnitt 421, der sich insbesondere von dem in Figur 2 erkennbaren proximalen Ende 42 der Übertragungsstange bis zum proximalen Ende des distalen Abschnitts 411 der Übertragungsstange 40 erstreckt. Der proximale Abschnitt 421 der Übertragungsstange 40 ist mittels einer Verbindungshülse 43 - insbesondere form- kraft- oder stoffschlüssig - mit dem distalen Abschnitt 411 der Übertragungsstange 40 gefügt. Der proximale Abschnitt 421 und die Verbindungshülse 43 sind von einem Isoliermantel 422 umschlossen. Das distale Ende des Isoliermantels 422 überlappt mit dem proximalen Ende der Isolierhülle 412 des distalen Abschnitt der Übertragungsstange 40.

Der proximale Abschnitt 421 der Übertragungsstange 40 und der Isoliermantel 422 sind insbesondere biegeflexibel und in Bezug auf Längskräfte und Torsion steif ausgebildet. Aufgrund der biegeflexiblen Ausbildung der Übertragungsstange 40 kann die Übertragungstange auch in einem gekrümmten Schaft 30 in Längsrichtung verschoben und um ihre Längsachse rotiert werden. Aufgrund der in Bezug auf Längskräfte und Torsion steifen Ausbildung der Übertragungsstange 40 kann die Übertragungsstange Kräfte und Drehmomente von der in den Figuren 1 und 2 dargestellten Handhabungseinrichtung zum Werkzeug 20 zu übertragen.

Das Werkzeug 20 umfasst ferner eine Verriegelungseinrichtung 48. Die Verriegelungseinrichtung 48 umfasst einen ringförmigen Abschnitt 480 und mehrere als konvexe Abschnitte an der Verriegelungseinrichtung 48 ausgebildete Riegel. Der ringförmige Abschnitt 480 und die Riegel 484 sind insbesondere einstückig ausgebildet. Der ringförmige Abschnitt 480 der Verriegelungseinrichtung 48 umschließt den distalen Abschnitt 411 und die Isolierhülle 412 der Übertragungsstange 40 unmittelbar proximal des Kragens 413 an der Isolierhülle 412.

Proximal des ringförmigen Abschnitts 480 der Verriegelungseinrichtung 48 umschließt ein Ring 44 die Isolierhülle 412 und den distalen Abschnitt 411 der Übertragungsstange 40. Der Ring 44 ist so angeordnet, dass zwischen dem Kragen 413 der Isolierhülle 412 und dem Ring 44 eine die Übertragungsstange 40 ringförmig umschließende Nut vorliegt, in der der ringförmige Abschnitt 480 der Verriegelungseinrichtung 48 spielarm und reibungsarm geführt ist. Die Verriegelungseinrichtung 48 kann somit um die Längsachse 29 des Werkzeugs 20 rotieren, ist aber in axialer Richtung präzise gerührt. Axiale Bewegungen der Übertragungsstange 40, wie sie im Vergleich der Figuren 19 bis 21 erkennbar sind, werden präzise in entsprechende axiale Bewegungen der Verriegelungseinrichtung 48 übertragen.

Die Riegel 484 an der Verriegelungseinrichtung 48 greifen in je einen axialen Abschnitt eines T-förmigen Schlitzes 274 in dem Verbindungsbauteil 27 ein. Somit ist die Verriegelungseinrichtung 48 hinsichtlich einer Rotation um die Längsachse 29 des Werkzeugs 20 durch die axialen Abschnitte der T-förmigen Schlitze 274 im Verbindungsbauteil 27, in die die Riegel 484 eingreifen, und hinsichtlich einer axialen Bewegung parallel zur Längsachse 29 des Werkzeugs 20 durch die umlaufende ringförmige Nut zwischen dem Kragen 413 an der Isolierhülle 412 und dem Ring 44, in die der ringförmige Abschnitt 480 der Verriegelungseinrichtung 48 eingreift, geführt.

Eine axiale Verschiebung der Übertragungsstange 40 parallel zur Achse 29 des Werkzeugs 20, wie sie im Vergleich der Figuren 19 bis 21 erkennbar ist, hat somit eine entsprechende Verschiebung der Verriegelungseinrichtung 48 zur Folge. Insbesondere befindet sich die Verriegelungseinrichtung 48 in der in Figur 19 dargestellten Montageposition 483, wenn sich die Maulteil 25, 26 in ihren in Figur 2 gezeigten überoffenen Positionen 253, 263 befinden. Die Verriegelungseinrichtung 48 befindet sich in einer ersten Arbeitsposition 481, wenn sich die Maulteile 25, 26 in ihren geschlossenen Positionen 251, 261 befinden. Die Verriegelungseinrichtung 48 befindet sich in einer zweiten Arbeitsposition 482, wenn sich die Maulteile 25, 26 in ihren offenen Positionen 252, 262 befinden. In allen Positionen 481, 482, 483 der Verriegelungseinrichtung 48 können die Übertragungsstange 40 und mit ihr die Gelenkeinrichtung 23 und die Maulteile 25, 26 relativ zum Verbindungsbauteil 27 und zum Schaft 30 frei um die Längsachse 29 des Werkzeugs 20 rotiert werden.

Nachfolgend wird mit Bezug auf die Figuren 22 bis 25 eine verriegelnde Wirkung der Riegel 484 der Verriegelungseinrichtung 48 auf eine mechanische Verbindung bzw. Kopplung des Werkzeugs 20 mit dem distalen Ende 31 eines Schafts 30 dargestellt. Die mechanische Verbindung bzw. Kopplung zwischen dem Werkzeug 20 und dem Schaft 30 erfolgt mittels des T-förmigen Schlitzes im proximalen Bereich des Verbindungsbauteils 27 und die Knagge 36 am distalen Ende des Schafts 30. In den Figuren 22 bis 25 sind deshalb nur das Verbindungsbauteil 27, das distale Ende des Schafts 30 mit der Knagge 36 und die Übertragungsstange 40 mit der Verriegelungseinrichtung 48 dargestellt.

In jeder der Figuren 22 bis 25 sind links ein Schnitt entlang einer Ebene A-A senkrecht zur Längsachse 29 des Werkzeugs 20 (vgl. Figuren 19 bis 21) und rechts jeweils zwei Draufsichten auf das Verbindungsbauteil 27, das distale Ende des Schafts 30 und die Übertragungsstange 40 gezeigt. Die Zeichenebenen der Darstellungen in den Figuren 22 bis 25 rechts sind jeweils senkrecht zur Schnittebene A-A, parallel zur Längsachse 29 des Werkzeugs 20 (vgl. Figuren 19 bis 21) und senkrecht zu den Schnitt- und Zeichenebenen der Figuren 19 bis 21. In den Figuren 22 bis 25 rechts ist jeweils die Lage der Schnittebene A-A angedeutet.

Die Draufsichten auf das Verbindungsbauteil 27, das distale Ende des Schafts 30 und die Übertragungsstange 40 sind in den Figuren 22 bis 25 rechts jeweils zweimal übereinander gezeigt. Jeweils oben sind die Konturen des Verbindungsbauteils 27 in durchgezogener Linie dargestellt, die Übertragungsstange 40 ist, soweit sie im Inneren des Verbindungsbauteils 27 oder des Schafts 30 verläuft, nur in gestrichelten Linien dargestellt. Jeweils unten ist die Übertragungsstange 40 - soweit sie nicht im Inneren des Schafts 30 angeordnet ist - in durchgezogenen Linien dargestellt, und das Verbindungsbauteil 27 ist lediglich in konturloser Schraffur angedeutet.

In den Figuren 22 bis 25 ist die T-förmige Gestalt jeweils eines Schlitzes 274 im proximalen Bereich des Verbindungsbauteils 27 erkennbar. Das Verbindungsbauteil 27 umfasst insbesondere zwei T-förmige Schlitze 274, die, bezogen auf die Längsachse 29 des Werkzeugs 20 (vgl. Figuren 19 bis 21) um 180° gegeneinander versetzt sind. Jeder T-förmige Schlitz im Verbindungsbauteil 27 umfasst einen axialen Abschnitt mit einem distalen Bereich 275 und einem proximalen Bereich 276 und einen umfänglichen Abschnitt 277. Der axiale Abschnitt 275, 276 erstreckt sich in axialer Richtung. Als distaler Bereich 275 des axialen Abschnitts des T-förmigen Schlitzes 274 wird der Bereich distal der Mündung des umfänglichen Abschnitts 277 in den axialen Abschnitt des T-förmigen Schlitzes 274 bezeichnet. Als proximaler Bereich 276 des axialen Abschnitts des T-förmigen Schlitzes 274 wird der Bereich proximal der Mündung des umfänglichen Abschnitts 277 in den axialen Abschnitt des T-förmigen Schlitzes 274 bezeichnet.

In den Figuren 22 bis 25 ist ferner der bereits genannte, jedoch in den Figuren 19 bis 21 nicht erkennbare axiale Schlitz 278 am Verbindungsbauteil 27 erkennbar, der sich vom distalen Rand 271 des Verbindungsbauteils 27 ausgehend in axialer Richtung erstreckt. Der Kragen 273 am distalen Rand 271 des Verbindungsbauteils 27 ist insbesondere von mehreren axialen Schlitzen 278 unterbrochen, beispielsweise von vier oder sechs Schlitzen. Die Schlitze 278 ermöglichen eine elastische Verformung des Verbindungsbauteils 27 an seinem distalen Ende beim Einsetzen in die Gelenkeinrichtung 23 bis zum Eingreifen des Kragens 273 in die Nut 237 an der Gelenkeinrichtung 23 (vgl. Figuren 19 bis 21). Die in den Figuren 19 bis 21 gezeigte Stützhülse 281, die den Kragen 273 nach der Montage in der umlaufenden Nut 237 hält, ist in den Figuren 22 bis 25 nicht gezeigt.

In den Figuren 22 bis 25 ist ferner ein axialer Schlitz 38 am distalen Ende des Schafts 30 erkennbar, dessen Funktion nachfolgend beschrieben ist, insbesondere mit Bezug auf die Figuren 24 und 25.

Bei den in den Figuren 22 und 23 dargestellten Situationen befindet sich die Verriegelungseinrichtung 48 in der auch in Figur 19 gezeigten Montageposition 483. Der Riegel 484 greift ausschließlich in den distalen Bereich 275 des axialen Abschnitts des T-förmigen Schlitzes 274 ein. Die Knagge 36 kann, wie in Figur 22 durch einen Pfeil angedeutet, durch eine axiale Bewegung und eine anschließende Drehbewegung des Schafts 30 relativ zum Verbindungsbauteil 27 durch den proximalen Bereich 276 in den umfänglichen Abschnitt 277 des T-förmigen Schlitzes 274 eingebracht werden.

Bei den in den Figuren 23, 24 und 25 dargestellten Situationen liegt die Knagge 36 im umfänglichen Abschnitt 277 des T-förmigen Schlitzes 274. Wenn die Knagge 36 im umfänglichen Abschnitt 277 des T-förmigen Schlitzes 274 liegt, ist durch Formschluss zwischen Knagge 36 und umfänglichem Abschnitt 277 des T-förmigen Schlitzes 274 eine mechanische Verbindung zwischen dem Verbindungsbauteil 27 und dem distalen Ende des Schafts 30 gebildet. Damit ist auch eine formschlüssige Verbindung zwischen dem gesamten Werkzeug 20 und dem Schaft 30 gebildet, die insbesondere Zugkräfte aufnimmt. Die T-förmigen Schlitze 274 am Verbindungsbauteil 27 und die Knaggen 36 am distalen Rand des Metallrohres 301 bilden somit Kupplungseinrichtungen oder sind Bestandteile von Kupplungseinrichtungen zur lösbaren mechanischen Kopplung des Verbindungsbauteils 27 mit dem distalen Ende 31 des Schafts 30.

Solange, wie in den Figuren 22 und 23 gezeigt, die Verriegelungseinrichtung 48 sich in der auch in Figur 19 gezeigten Montageposition 483 befindet, kann die Knagge 36 durch eine Drehbewegung und eine anschließende axiale Bewegung des Schafts 30 relativ zu dem Verbindungsbauteil 27 wieder aus dem T-förmigen Schlitz 274 im Verbindungsbauteil 27 entfernt werden.

Bei der in Figur 24 dargestellten Situation befindet sich die Verriegelungseinrichtung 48 in der auch in Figur 20 gezeigten zweiten Arbeitsposition 482. Bei der in Figur 25 gezeigten Situation befindet sich die Verriegelungseinrichtung 48 in der auch in Figur 21 gezeigten ersten Arbeitsposition 481. Sowohl in der ersten Arbeitsposition 481 als auch in der zweiten Arbeitsposition 482 der Verriegelungseinrichtung 48 greift die Verriegelungseinrichtung 48 in radialer Richtung durch den Schlitz 38 am distalen Ende des Schafts 30 hindurch. Insbesondere greift der Riegel 484 durch den Schlitz 38 hindurch nach radial außen.

In beiden Arbeitspositionen 481, 482 der Verriegelungseinrichtung 48 liegt der Riegel 484 der Verriegelungseinrichtung 48 nicht mehr ausschließlich im distalen Bereich 275 des T-förmigen Schlitzes 274, sondern zumindest teilweise im Bereich der Mündung des umfänglichen Abschnitts 277 des T-förmigen Schlitzes 274 in den axialen Abschnitt 275, 276. Damit hält bzw. blockiert der Riegel 484 die Knagge 36 im umfänglichen Abschnitt 277 des T-förmigen Schlitzes 274. Somit verriegelt die Verriegelungseinrichtung 48, insbesondere der Riegel 484, in den in den Figuren 20, 21, 24 und 25 gezeigten Arbeitspositionen 481, 482 die mechanische Verbindung bzw. Kopplung des Werkzeugs 20 mit dem distalen Ende des Schafts 30.

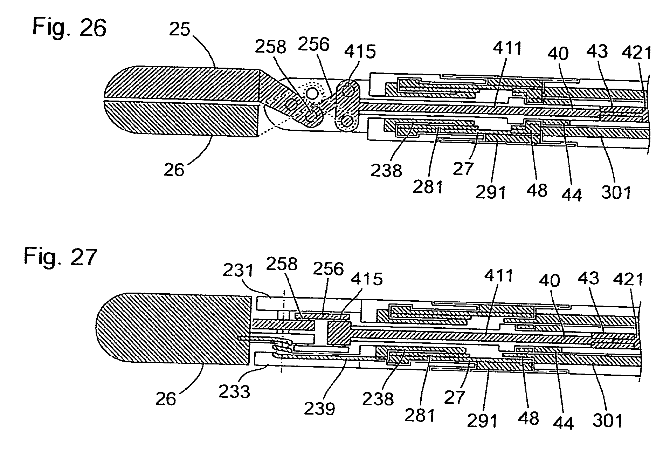

Der anhand der Figuren 19 bis 21 gezeigte Aufbau des Werkzeugs 20 eignet sich für ein bipolares mikroinvasiv-chirurgisches Instrument, bei dem zwischen den Maulteilen 25, 26 eine hochfrequente Wechselspannung angelegt wird, um durch die Maulteile 25, 26 gegriffenes oder zerschnittenes Gewebe durch Stromfluss zu veröden, insbesondere Eiweiß zu koagulieren. Nachfolgend wird die Kontaktierung der Maulteile 25, 26 bzw. die Zuführung elektrischen Stroms und elektrischer Leistung zu den Maulteilen 25, 26 anhand der Figuren 26 und 27 beschrieben.

In den Figuren 26 und 27 ist das Werkzeug 20 mit dem distalen Ende 31 des Schafts 30 in schematischen Schnittdarstellungen gezeigt. Die Schnittebene der Figur 26 entspricht den Schnittebenen der Figuren 19 bis 21. Die Schnittebene der Figur 27 ist senkrecht zu den Schnittebenen der Figuren 19 bis 21 und 26, parallel zu den Zeichenebenen der Darstellungen in den Figuren 22 bis 25 rechts und parallel zur Längsachse 29 des Werkzeugs 20.

In den Figuren 26 und 27 sind alle elektrisch leitfähigen Elemente, die mit dem ersten Maulteil 25 elektrisch leitfähig verbunden sind, mit einer Schraffur von links unten nach rechts oben versehen. Alle Elemente, die mit dem zweiten Maulteil 26 elektrisch leitfähig verbunden sind, sind mit einer Schraffur von rechts unten nach links oben versehen. Elemente, die elektrisch isolierende Materialien aufweisen, sind ebenso wie Hohlräume nicht schraffiert und - von den Holmen 231, 233 abgesehen - nicht mit Bezugszeichen versehen. Um die elektrisch leitfähige Verbindung zu den Maulteilen, insbesondere zum zweiten Maulteil 26 darzustellen, weicht die Figur 27 im Bereich links der Bildmitte bzw. distal der Gelenke 415, 416 am distalen Ende der Übertragungsstange 40 von einer Schnittdarstellung ab. In diesem distalen Bereich entspricht die Figur 27 eher einer Draufsicht.

Das erste Maulteil 25 ist über den proximalen Abschnitt 421 der Übertragungsstange 40, die Verbindungshülse 43, den distalen Abschnitt 411 der Übertragungsstange 40, das Gelenk 415, das erste Pleuel 256 und das Gelenk 258 am ersten Maulteil 25 elektrisch kontaktiert. Das zweite Maulteil 26 ist über das Metallrohr 301 des Schafts 30, die in keiner der beiden Schnittebenen der Figuren 26 und 27 liegende Knagge 36 (vgl. Figuren 19 und 22 bis 25), die Verriegelungseinrichtung 48, das Verbindungsbauteil 27, die Stützhülse 281, das Kontaktbauteil 238 und ein nur in Figur 27 dargestelltes elastisches, elektrisch leitfähiges Bauteil 239 kontaktiert.

Das elastische elektrisch leitfähige Bauteil 239 wird insbesondere durch einen metallischen Draht gebildet, der einen helikalen Abschnitt an der Welle 232 aufweist. Das proximale Ende des elastischen elektrisch leitfähigen Bauteils 239 ist mit dem distalen Ende des Kontaktbauteils 238 verbunden, beispielsweise gesteckt, geklemmt, gelötet oder verschweißt. Insbesondere ist das proximale Ende des elastischen elektrisch leitfähigen Bauteils 239 abweichend von der Darstellung in Figur 27 in das Lumen eines mit dem Kontaktbauteil 238 verschweißten oder verlöteten Rohres gesteckt. Eine leichte Krümmung des proximalen Endes des elastischen elektrisch leitfähigen Bauteils 239 kann eine elastische Klemmung des proximalen Endes des elastischen elektrisch leitfähigen Bauteils 239 in dem Rohr und damit einen zuverlässigen Kontakt bewirken. Das distale Ende des elastischen elektrisch leitfähigen Bauteils 239 ist elektrisch leitfähig mit dem zweiten Maulteil 26 verbunden, beispielsweise geklemmt, gesteckt, verlötet oder verschweißt.

Der proximale Abschnitt des elastischen elektrisch leitfähigen Bauteils 239 ist insbesondere in einem der Holme 231, 233 der Gelenkeinrichtung 23 angeordnet. Ein distaler Abschnitt des elastischen elektrisch leitfähigen Bauteils 239 ist insbesondere in einem elektrisch isolierenden Abschnitt des zweiten Maulteils 26 angeordnet, der das Lager an der Welle 232 und das Gelenk 268 zum zweiten Pleuel 266 umfasst.

Bei den oben dargestellten Ausführungsformen ist die Übertragungsstange 40 sowohl zur Übertragung einer translatorischen Bewegung in einer Richtung parallel zum Schaft 30 bzw. zu dessen Längsachse und einer entsprechenden Zug- und/oder Schubkraft als auch zur Übertragung einer rotatorischen Bewegung und eines entsprechenden Drehmoments zum Werkzeug 20 ausgebildet. Abweichend von der Darstellung anhand der Figuren kann das Werkzeug 20 am distalen Ende 31 des Schafts 30 lediglich um die Längsachse des distalen Endes 31 des Schafts 30 oder um eine andere Achse rotierbar sein. Beispielsweise umfasst das Werkzeug 20 einen fingerförmigen oder anderen Manipulator oder eine Elektrode in Haken- oder Schlaufenform oder in anderer Gestalt. In diesem Fall ist eine Ausbildung der Übertragungsstange 40 für die Übertragung einer translatorischen Bewegung in einer Richtung parallel zum Schaft 30 und für die Übertragung einer entsprechenden Kraft nur insoweit erforderlich, als sie zum Bewegen der Verriegelungseinrichtung 48 erforderlich ist. Alternativ kann ein fingerförmiger oder anderer Manipulator oder eine Elektrode in Haken- oder Schlaufenform oder in anderer Gestalt nicht nur rotiert sondern zusätzlich mittels einer Längsbewegung der Übertragungsstange 40 parallel zum Schaft 30 bzw. zu dessen Längsachse bewegbar sein. Beispielsweise ist ein hakenförmiger Manipulator ausgebildet, um bei einer Bewegung im Wesentlichen parallel zur Längsachse des Schafts 30 nach proximal einen Gegenstand zwischen dem hakenförmigen Manipulator und einem nicht in Längsrichtung verschiebbaren Abschnitt des Werkzeugs zu klemmen.

### Bezugszeichen

- 10: Mikroinvasiv-chirurgisches Instrument
- 11: distales Ende des mikroinvasiv-chirurgischen Instruments 10
- 12: proximales Ende des mikroinvasiv-chirurgischen Instruments 10
- 19: Richtung des Zusammenbaus
- 20: Werkzeug des mikroinvasiv-chirurgischen Instruments 10
- 21: distales Ende des Werkzeugs 20
- 22: proximales Ende des Werkzeug 20
- 226: Knagge am proximalen Ende 22 des Werkzeugs 20
- 23: Gelenkeinrichtung
- 231: erster Holm der Gelenkeinrichtung 23
- 232: Welle an der Gelenkeinrichtung 23
- 233: zweiter Holm der Gelenkeinrichtung 23
- 234: Führungsstift an Gelenkeinrichtung 23
- 235: Ring
- 236: Nut für Drehzapfen 264 am zweiten Maulteil 26
- 237: Nut an der Gelenkeinrichtung 23
- 238: Kontaktbauteil
- 239: elastisches elektrische leitfähiges Bauteil
- 25: erstes Maulteil des Werkzeugs 20
- 251: geschlossene Position des ersten Maulteils 25
- 252: offene Position des ersten Maulteils 25
- 253: überoffene Position des ersten Maulteils 25
- 254: Führungsschlitz in erstem Maulteil 25 für Führungsstift 234 an Gelenkeinrichtung 23
- 256: erstes Pleuel zum ersten Maulteil 25
- 258: Gelenk zwischen dem ersten Maulteil 25 und dem ersten Pleuel 256
- 259: Schwenkachse des ersten Maulteils 25
- 26: zweites Maulteil des Werkzeugs 20
- 261: geschlossene Position des zweiten Maulteils 26
- 262: offene Position des zweiten Maulteils 26
- 263: überoffene Position des zweiten Maulteils 26
- 264: Führungsschlitz in zweitem Maulteil 26 für Führungsstift 234 an Gelenkeinrichtung 23
- 265: Drehzapfen des zweiten Maulteils 26
- 266: zweites Pleuel zum zweiten Maulteil 26
- 267: Mitnahmeschlitz am zweiten Maulteil 26 für Steuerzapfen 466 an Getriebeplatte 46
- 268: Gelenk zwischen dem zweiten Maulteil 26 und dem zweiten Pleuel 266
- 269: Schwenkachse des zweiten Maulteils 26
- 27: Verbindungsbauteil
- 271: distales Ende des Verbindungsbauteils 27
- 272: proximales Ende des Verbindungsbauteils 27
- 273: Kragen am distalen Ende 271 des Verbindungsbauteils 27
- 274: T-förmiger Schlitz am proximalen Ende 272 des Verbindungsbauteils 27
- 275: distaler Bereich des axialen Abschnitts des T-förmigen Schlitzes 274
- 276: proximaler Bereich des axialen Abschnitts des T-förmigen Schlitzes 274
- 277: umfänglicher Abschnitt des T-förmigen Schlitzes 274
- 278: axialer Schlitz am Verbindungsbauteil
- 279: Nut am distalen Ende 271 des Verbindungsbauteils 27
- 281: Stützhülse
- 29: Längsachse des Werkzeugs 20
- 291: Handhabungsring am Werkzeug 20
- 292: Isolierhülle

- 30: Schaft des mikroinvasiv-chirurgischen Instruments 10
- 301: Metallrohr des Schafts 30
- 302: Isoliermantel des Schafts 30
- 303: Stützrohr am distalen Ende 31 des Schafts 30
- 304: Bajonetthülse
- 306: L-förmiger Schlitz in der Bajonetthülse 304
- 31: distales Ende des Schafts 30
- 32: proximales Ende des Schafts 30
- 34: Kanal im Schaft 30
- 35: umlaufende Nut nahe dem proximalen Ende 32 des Schafts 30
- 36: Knagge am distalen Ende 31 des Schafts 30
- 38: axialer Schlitz im distalen Ende 31 des Schafts 30

- 40: Übertragungsstange des mikroinvasiv-chirurgischen Instruments 10
- 41: distales Ende der Übertragungsstange 40
- 411: distaler Abschnitt der Übertragungsstange 40
- 412: Isolierhülle am distalen Ende 41 der Übertragungsstange 40
- 413: Kragen an der Isolierhülle 412
- 415: Gelenk zwischen dem distalem Ende 41 der Übertragungsstange 40 und dem ersten Pleuel 256
- 416: Gelenk zwischen dem distalem Ende 41 der Übertragungsstange 40 und dem zweiten Pleuel 266
- 42: proximales Ende der Übertragungsstange 40
- 421: proximaler Abschnitt der Übertragungsstange 40
- 422: Isoliermantel an der Übertragungsstange 40
- 43: Verbindungshülse zwischen proximalem Abschnitt 421 und distalem Abschnitt 411
- 44: Ring an der Übertragungsstange 40
- 45: Klaue am proximalen Ende 42 der Übertragungsstange 40
- 46: Platte am distalen Ende 41 der Übertragungsstange 40
- 464: Führungsschlitz in der Platte 47
- 465: Steuerzapfen an Platte 46 für erstes Maulteil 25
- 466: Steuerzapfen an Platte 46 für zweites Maulteil 26
- 48: Verriegelungseinrichtung
- 480: ringförmiger Abschnitt der Verriegelungseinrichtung 48
- 481: erste Arbeitsposition der Verriegelungseinrichtung 48
- 482: zweite Arbeitsposition der Verriegelungseinrichtung 48
- 483: Montageposition der Verriegelungseinrichtung 48
- 484: Riegel an der Verriegelungseinrichtung
- 486: Zapfen an der Verriegelungseinrichtung 48
- 487: Kopf am radial äußeren Ende des Zapfens 486

- 50: Handhabungseinrichtung des mikroinvasiv-chirurgischen Instruments 10
- 51: distales Ende der Handhabungseinrichtung 50
- 52: proximales Ende der Handhabungseinrichtung 50
- 538: Entriegelungsknopf am Riegel 530
- 57: Drehrad
- 578: Achse
- 58: erstes Griffteil der Handhabungseinrichtung 50
- 59: zweites Griffteil der Handhabungseinrichtung 50
- 591: erste Arbeitsposition des zweiten Griffteils 59
- 592: zweite Arbeitsposition des zweiten Griffteils 59
- 593: Montageposition des zweiten Griffteils 59

## Patentansprüche

1. **Werkzeug** (20), das mit einem distalen Ende (31) eines Schafts (30) für ein mikroinvasivchirurgisches Instrument (10) lösbar mechanisch koppelbar ist, mit:
einer **Gelcnkeinrichtung** (23), an der ein Maulteil (25, 26) oder eine andere Wirkeinrichtung befestigt ist;
einem **Verbindungsbauteil** (27), das mit der Gelenkeinrichtung (23) rotierbar mechanisch verbunden ist, und das eine Kupplungseinrichtung (226; 274) zur lösbaren mechanischen Kopplung mit einem distalen Ende (31) eines Schafts (30) aufweist,
**wobei die Kupplungseinrichtung (226; 274) als Bajoncttkupplung ausgebildet** ist;
einer **Übertragungsstange** (40) zum Übertragen zumindest entweder einer Kraft oder eines Drehmoments von einem proximalen Ende (32) eines mit dem Werkzeug (20) lösbar mechanisch gekoppelten Schafts (30) zu dem Maulteil (25, 26) oder der anderen Wirkeinrichtung,
einer **Verriegelungseinrichtung** (48), die mit der Übertragungsstange (40) derart mechanisch gekoppelt ist, dass die Verriegelungseinrichtung relativ zu der Übertragungsstange rotierbar, aber nicht axial verschiebbar ist, wobei die Verriegelungseinrichtung in dem Verbindungsbauteil (27) derart mechanisch gelagert ist, dass sie relativ zum Verbindungsbauteil (27) axial verschiebbar, aber nicht rotierbar ist,
wobei die **Verriegelungseinrichtung** (48) in axialer Richtung zwischen einer Montageposition (483) und einer Arbeitsposition (481,482) **verschiebbar** ist, wobei in der Montageposition (483) der Verriegelungseinrichtung (48) das Verbindungsbauteil (27) mit einem distalen Ende (31) eines Schafts (30) verbindbar und von diesem lösbar ist, und wobei in der Arbeitsposition (481, 482) der Verriegelungseinrichtung (48) eine mechanische Verbindung des Werkzeugs (20) mit einem distalen Ende (31) eines Schafts (30) verriegelt ist.

2. Werkzeug (20) nach Anspruch 1, bei dem die Verriegelungseinrichtung (48) einen **ringförmigen Abschnitt** (480) umfasst, der in eine umlaufende **Nut** an der Übertragungsstange (40) eingreift.

3. Werkzeug (20) nach einem der vorangehenden Ansprüche, bei dem die **Verriegelungseinrichtung** (48) zumindest entweder ein **elektrisch isolierendes** Material aufweist oder gegenüber der Übertragungsstange (40) elektrisch isoliert ist.

4. Werkzeug (20) nach einem der vorangehenden Ansprüche, bei dem die Gelenkeinrichtung (23) ein elektrisch isolierendes Material aufweist, ferner mit:
einem elektrisch leitfähigen **Leiterbauteil** (239), das zumindest teilweise in der Gelenkeinrichtung (23) angeordnet ist, zur elektrischen Kontaktierung eines Maulteils (26) oder einer anderen Wirkeinrichtung, das bzw. die gegenüber der Übertragungsstange (40) elektrisch isoliert ist.

5. Werkzeug (20) nach einem der vorangehenden Ansprüche, bei dem die Kupplungseinrichtung als Bajonettkupplung mit einer **Knagge** (226) ausgebildet ist, die Verriegelungseinrichtung (48) einen **konvexen Abschnitt** (487) umfasst,
wobei die Knagge (226) und der konvexe Abschnitt (487) an der Verriegelungseinrichtung (48) in axialer Richtung und in umfänglicher Richtung beabstandet und ausgebildet sind, um in eine **Nut** oder einen Schlitz (306) an einem distalen Ende (31) eines Schafts (30) einzugreifen.

6. Werkzeug (20) nach einem der vorangehenden Ansprüche, bei der die Verriegelungseinrichtung (48) ausgebildet und angeordnet ist, um in der Arbeitsposition (481,482) durch **Eingriff in** einen sich in axialer Richtung erstreckenden Abschnitt (306) einer **Nut** oder eines Schlitzes an einem distalen Ende (31) eines Schafts (30) eine Rotation der Kupplungseinrichtung (226) relativ zu dem distalen Ende (31) des Schafts (30) zu hernmen.

7. Werkzeug (20) nach einem der Ansprüche 1 bis 4, bei der die Verriegelungseinrichtung (48) in eine Nut bzw. einen Schlitz (275, 276, 277) an der als **Bajonctt**kupplung ausgebildeten Kupplungseinrichtung eingreift.

8. Werkzeug (20) nach dem vorangehenden Anspruch, bei dem
die Nut bzw der Schlitz eine **T-förmige** Gestalt mit einem axialen Abschnitt (275, 276), der sich in axialer Richtung erstreckt, und einem umfänglichen Abschnitt (277), der sich in umfänglicher Richtung erstreckt, aufweist, wobei ein proximaler Bereich (276) des axialen Abschnitts proximal einer Mündung des umfänglichen Abschnitts (277) in den axialen Abschnitt angeordnet ist, und wobei ein distaler Bereich (275) des axialen Abschnitts distal einer Mündung des umfängliche Abschnitts (277) in den axialen Abschnitt angeordnet ist;
die Verriegelungseinrichtung (48) in der **Montageposition** (483) nur in den distalen Bereich (275) des axialen Abschnitts der Nut bzw. des Schlitzes eingreift, so dass eine Knagge (36) an einem mit dem Werkzeug (20) zu verbindenden Schaft (30) durch den proximalen Bereich (276) des axialen Abschnitts in den umfänglichen Abschnitt (277) der Nut bzw. des Schlitzes eingeführt werden kann;
die Verriegelungseinrichtung (48) in der **Arbeitsposition** (481, 482) zumindest teilweise die Mündung des umfänglichen Abschnitts (277) der Nut bzw. des Schlitzes in den axialen Abschnitt (275, 276) blockiert, so dass eine im umfänglichen Abschnitt (277) der Nut bzw. des Schlitzes angeordnete Knagge (36) an einem mit dem Werkzeug (20) verbundenen Schaft (30) durch die Verriegelungseinrichtung (48) im umfänglichen Abschnitt (277) gehalten wird.

9. Werkzeug (20) nach einem der vorangehenden Ansprüche, bei dem
das Maulteil (25) von einem weiteren Maulteil (26) **elektrisch isoliert** ist;
das weitere Maulteil (26) elektrisch leitfähig mit einem **Kontaktbauteil** (238) verbunden ist, das mit der Gelenkeinrichtung (23) mechanisch starr verbunden ist;
eine proximale **Stirnfläche** des Kontaktbauteils (238) an einer mit dem Verbindungsbauteil (27) mechanisch starr verbundenen Kontaktfläche anliegt;
das Kontaktbauteil (238) und die mit dem Verbindungsbauteil (27) mechanisch starr verbundene Kontaktfläche vorgesehen und ausgebildet sind, um eine elektrisch **leitfähige Verbindung** zwischen einem mit dem Werkzeug verbundenen Schaft und dem weiteren Maulteil (26) zu bilden.

10. Werkzeug (20) nach einem der Ansprüche 1 bis 8, bei dem das Verbindungsbauteil (27) nahe seinem distalen Ende (271) einen **Kragen** (273) aufweist, der von mehreren im Wesentlichen axialen Schlitzen (278) unterbrochen ist und in eine korrespondierende **Nut** (237) an der Gelenkeinrichtung (23) eingreift, ferner mit:
einer **Stützeinrichtung** (281), die ausgebildet ist, um eine radiale Verformung des Kragens (273) zu hemmen und den Kragen (273) in der Nut (237) zu halten.

11. Werkzeug (20) nach dem vorangehenden Anspruch, bei dem die Stützeinrichtung als Stützhülse (281) ausgebildet ist, ferner mit:
einem **Kontaktbauteil** (238) mit einer Kontaktfläche, die an der Stützhülse (281) anliegt, wobei das Kontaktbauteil (238) ausgebildet und angeordnet ist, zusammen mit der Gelenkeinrichtung (23) gegenüber dem Verbindungsbauteil rotierbar zu sein.

12. Werkzeug (20) nach einem der vorangehenden Ansprüche, wobei das Maulteil (25, 26) gekrümmt ist.

13. **Mikroinvasiv-chirurgisches Instrument** (10), mit:
einem Schaft (30) mit einem proximalen Ende (32), das mit einer Handhabungseinrichtung (50) koppelbar oder gekoppelt ist, und einem distalen Ende (31);
einem Werkzeug (20) nach einem der vorangehenden Ansprüche, das mit dem distalen Ende (31) des Schaft (30) lösbar mechanisch koppelbar ist.

14. Mikroinvasiv-chirurgisches Instrument nach dem vorangehenden Anspruch in Rückbezug auf Anspruch 5, bei dem das distale Ende (31) des Schafts (30) einen Bajonettkupplungsbereich (304) mit einer **L-förmige Nut** oder einen L-förmigen Schlitz (306, 307) aufweist.

## Claims

1. Tool (20) that can be mechanically coupled in a releasable manner to a distal end (31) of a shank (30) for a micro-invasive surgical instrument (10), with:
a hinge device (23); on which a jaw part (25, 26) or another effecting device is secured;
a connecting component (27), which is mechanically connected in a rotatable manner to the hinge device (23) and which has a coupling device (226; 274) for the releasable mechanical coupling to a distal end (31) of a shank (30),
wherein the coupling device (226; 274) is formed as a bayonet coupling;
a transmission rod (40) for transmitting at least either a force or a torque from a proximal end (32) of a shank (30), mechanically coupled in a releasable manner to the tool (20), to the jaw part (25, 26) or to the other effecting device;
a locking device (48), which is mechanically coupled to the transmission rod (40) in such a way that the locking device is rotatable relative to the transmission rod but is not axially movable, wherein the ocking device is mechanically mounted in the connecting component (27) in such a way that it is axially movable relative to the connecting component (27) but is not rotatable,
wherein the locking device (48) is movable in the axial direction between an installation position (483) and a working position (481, 482), wherein the connecting component (27), in the installation position (483) of the locking device (48), is connectable to a distal end (31) of a shank (30) and releasable therefrom, and wherein a mechanical connection of the tool (20) to a distal end (31) of a shank (30) is locked in the working position (481, 482) of the locking device (48).

2. Tool (20) according to Claim 1, in which the locking device (48) comprises an annular portion (480), which engages in a circumferential groove on the transmission rod (40).

3. Tool (20) according to one of the preceding claims, in which the locking device (48) at least either has an electrically insulating material or is electrically insulated from the transmission rod (40).

4. Tool (20) according to one of the preceding claims, in which the hinge device (23) has an electrically insulating material, moreover with:
an electrically conductive conductor component (239), which is arranged at least partially in the hinge device (23) for electrical contacting of a jaw part (26) or another effecting device that is electrically insulated from the transmission rod (40).

5. Tool (20) according to one of the preceding claims, in which
the coupling device is formed as a bayonet coupling with a catch (226),
the locking device (48) comprises a convex portion (487),
wherein the catch (226) and the convex portion (487) on the locking device (48) are spaced apart in the axial direction and in the circumferential direction and are designed to engage in a groove or a slit (306) on a distal end (31) of a shank (30).

6. Tool (20) according to one of the preceding claims, in which the locking device (48) is designed and arranged in order to engage, in the working position (481, 482), in an axially extending portion (306) of a groove or of a slit on a distal end (31) of a shank (30) in order thereby to inhibit a rotation of the coupling device (226) relative to the distal end (31) of the shank (30).

7. Tool (20) according to one of Claims 1 to 4, in which the locking device (48) engages in a groove or a slit (275, 276, 277) on the coupling device designed as the bayonet coupling.

8. Tool (20) according to the preceding claim, in which
the groove or the slit has a T-shaped configuration with an axial portion (275, 276), which extends in an axial direction, and with a circumferential portion (277), which extends in a circumferential direction, wherein a proximal area (276) of the axial portion is arranged proximally from an opening of the circumferential portion (277) into the axial portion, and wherein a distal area (275) of the axial portion is arranged distally from an opening of the circumferential portion (277) into the axial portion;
the locking device (48), in the installation position (483), engages only in the distal area (275) of the axial portion of the groove or slit, such that a catch (36) on a shank (30) to be connected to the tool (20) can be inserted through the proximal area (276) of the axial portion into the circumferential portion (277) of the groove or the slit;
the locking device (48), in the working position (481, 482), at least partially blocks the opening of the circumferential portion (277) of the groove or slit into the axial portion (275, 276), such that a catch (36) arranged in the circumferential portion (277) of the groove or slit is held on a shank (30), connected to the tool (20), by the locking device (48) in the circumferential portion (277).

9. Tool (20) according to one of the preceding claims, in which
the jaw part (25) is electrically insulated from a further jaw part (26);
the further jaw part (26) is electrically conductively connected to a contact component (238), which is mechanically rigidly connected to the hinge device (23);
a proximal front face of the contact component (238) bears on a contact face that is mechanically rigidly connected to the connecting component (27);
the contact component (238) and the contact face mechanically rigidly connected to the connecting component (27) are provided and designed to form an electrically conductive connection between a shank, connected to the tool, and the further jaw part (26).

10. Tool (20) according to one of Claims 1 to 8, in which the connecting component (27) has, near its distal end (271), a collar (273) which is interrupted by several substantially axial slits (278) and which engages in a corresponding groove (237) on the hinge device (23), moreover with:
a supporting device (281), which is designed to inhibit a radial deformation of the collar (273) and to hold the collar (273) in the groove (237).

11. Tool (20) according to the preceding claim, in which the supporting device is designed as a supporting sleeve (281), moreover with:
a contact component (238) with a contact face which bears on the supporting sleeve (281), wherein the contact component (238) is designed and arranged to be rotatable, together with the hinge device (23), with respect to the connecting component.

12. Tool (20) according to one of the preceding claims, wherein the jaw part (25, 26) is curved.

13. Micro-invasive surgical instrument (10), with:
a shank (30) with a proximal end (32), which is or can be coupled to a handling device (50), and with a distal end (31);
a tool (20) according to one of the preceding claims, which can be mechanically coupled in a releasable manner to the distal end (31) of the shank (30).

14. Micro-invasive surgical instrument according to the preceding claim in back-reference to Claim 5, in which the distal end (31) of the shank (30) has a bayonet coupling area (304) with an L-shaped groove or an L-shaped slit (306, 307).

## Revendications

1. Outil (20) pouvant être accouplé mécaniquement de manière amovible avec une extrémité distale (31) d'une tige (30) pour un instrument chirurgical micro-invasif (10), comprenant :
un dispositif articulé (23) auquel est fixée une partie formant bec (25, 26) ou un autre dispositif actif ;
un composant de liaison (27) qui est relié mécaniquement en rotation avec le dispositif articulé (23) et qui possède un dispositif d'accouplement (226 ; 274) servant à l'accouplement mécanique amovible avec une extrémité distale (31) d'une tige (30),
le dispositif d'accouplement (226 ; 274) étant réalisé sous la forme d'un coupleur à baïonnette ;
une baguette de transmission (40) destinée à transmettre au moins soit une force, soit un couple, depuis une extrémité proximale (32) d'une tige (30) accouplée mécaniquement de manière amovible avec l'outil (20) vers la partie formant bec (25, 26) ou l'autre dispositif actif,
un dispositif de verrouillage (48) qui est accouplé mécaniquement avec la baguette de transmission (40) de telle sorte que le dispositif de verrouillage peut effectuer une rotation par rapport à la baguette de transmission, mais ne peut pas coulisser dans le sens axial, le dispositif de verrouillage étant logé mécaniquement dans le composant de liaison (27) de telle sorte qu'il peut coulisser dans le sens axial par rapport au composant de liaison (27), mais pas effectuer une rotation,
le dispositif de verrouillage (48) pouvant coulisser dans le sens axial entre une position de montage (483) et une position de travail (481, 482), le composant de liaison (27) pouvant être relié à une extrémité distale (31) d'une tige (30) et détaché de celle-ci dans la position de montage (483) du dispositif de verrouillage (48) et, dans la position de travail (481, 482) du dispositif de verrouillage (48), une liaison mécanique de l'outil (20) avec une extrémité distale (31) d'une tige (30) étant verrouillée.

2. Outil (20) selon la revendication 1, avec lequel le dispositif de verrouillage (48) comprend une portion de forme annulaire (480) qui vient en prise dans une rainure circonférentielle sur la baguette de transmission (40).

3. Outil (20) selon l'une des revendications précédentes, avec lequel le dispositif de verrouillage (48) soit possède au moins un matériau électriquement isolant, soit est isolé électriquement par rapport à la baguette de transmission (40).

4. Outil (20) selon l'une des revendications précédentes, avec lequel le dispositif articulé (23) possède un matériau électriquement isolant, comprenant en outre :
un composant conducteur (239) électriquement conducteur qui est disposé au moins partiellement dans le dispositif articulé (23) et sert à établir le contact électrique avec une partie formant bec (26) ou un autre dispositif actif qui est isolé électriquement par rapport à la baguette de transmission (40).

5. Outil (20) selon l'une des revendications précédentes, avec lequel
le dispositif d'accouplement est réalisé sous la forme d'un coupleur à baïonnette muni d'un taquet (226),
le dispositif de verrouillage (48) comprend une portion convexe (487),
le taquet (226) et la portion convexe (487) étant espacés dans le sens axial et dans le sens circonférentiel sur le dispositif de verrouillage (48) et étant configurés pour venir en prise dans une rainure ou une fente (306) au niveau d'une extrémité distale (31) d'une tige (30).

6. Outil (20) selon l'une des revendications précédentes, avec lequel le dispositif de verrouillage (48) est configuré et disposé pour, en position de travail (481, 482), en venant en prise dans une portion (306) d'une rainure ou d'une fente qui s'étend dans le sens axial sur une extrémité distale (31) d'une tige (30), empêcher une rotation du dispositif d'accouplement (226) par rapport à l'extrémité distale (31) de la tige (30).

7. Outil (20) selon l'une des revendications 1 à 4, avec lequel le dispositif de verrouillage (48) vient en prise dans une rainure ou une fente (275, 276, 277) sur le dispositif d'accouplement réalisé sous la forme d'un coupleur à baïonnette.

8. Outil (20) selon la revendication précédente, avec lequel
la rainure ou la fente présente une configuration en forme de T dotée d'une portion axiale (275, 276) qui s'étend dans le sens axial et une portion circonférentielle (277) qui s'étend dans une direction circonférentielle, une zone proximale (276) de la portion axiale étant disposée de manière proximale à un débouché de la portion circonférentielle (277) dans la portion axiale, et une zone distale (275) de la portion axiale étant disposée de manière distale à un débouché de la portion circonférentielle (277) dans la portion axiale ;
le dispositif de verrouillage (48) en position de montage (483) ne vient en prise que dans la zone distale (275) de la portion axiale de la rainure ou de la fente, de sorte qu'un taquet (36) sur une tige (30) à relier à l'outil (20) peut être introduit dans la portion circonférentielle (277) de la rainure ou de la fente à travers la zone proximale (276) de la portion axiale ;
le dispositif de verrouillage (48) en position de travail (481, 482) bloque au moins partiellement le débouché de la portion circonférentielle (277) de la rainure ou de la fente dans la portion axiale (275, 276), de sorte qu'un taquet (36) disposé dans la portion circonférentielle (277) de la rainure ou de la fente est maintenu par le dispositif de verrouillage (48) dans la portion circonférentielle (277) sur une tige (30) reliée à l'outil (20).

9. Outil (20) selon l'une des revendications précédentes, avec lequel
la partie formant bec (25) est isolée électriquement d'une partie formant bec supplémentaire (26) ;
la partie formant bec supplémentaire (26) est reliée de manière électriquement conductrice à un composant de contact (238) qui est relié de manière mécaniquement rigide au dispositif articulé (23) ;
une face frontale proximale du composant de contact (238) repose sur une surface de contact reliée de manière mécaniquement rigide au composant de liaison (27) ;
le composant de contact (238) et la surface de contact reliée de manière mécaniquement rigide au composant de liaison (27) sont conçus et configurés pour former une liaison électriquement conductrice entre une tige reliée à l'outil et la partie formant bec supplémentaire (26).

10. Outil (20) selon l'une des revendications 1 à 8, avec lequel le composant de liaison (27) possède à proximité de son extrémité distale (271) une collerette (273) qui est interrompue par plusieurs fentes (278) sensiblement axiales et vient en prise dans une rainure (237) correspondante dans le dispositif articulé (23), comprenant en outre :
un dispositif support (281) qui est configuré pour empêcher une déformation radiale de la collerette (273) et maintenir la collerette (273) dans la rainure (237).

11. Outil (20) selon la revendication précédente, avec lequel le dispositif support est réalisé sous la forme d'une douille support (281), comprenant en outre :
un composant de contact (238) doté d'une face de contact qui repose sur la douille support (281), le composant de contact (238) étant configuré et disposé pour pouvoir tourner conjointement avec le dispositif articulé (23) par rapport au composant de liaison.

12. Outil (20) selon l'une des revendications précédentes, avec lequel la partie formant bec (25, 26) est courbée.

13. Instrument chirurgical micro-invasif (10) comprenant :
une tige (30) dotée d'une extrémité proximale (32) qui peut être ou est accouplée avec un dispositif de manipulation (50), et une extrémité distale (31) ;
un outil (20) selon l'une des revendications précédentes qui peut être accouplé mécaniquement de manière amovible avec l'extrémité distale (31) de la tige (30).

14. Instrument chirurgical micro-invasif selon la revendication précédente en référence à la revendication 5, avec lequel l'extrémité distale (31) de la tige (30) présente une zone d'accouplement à baïonnette (304) dotée d'une rainure en forme de L ou d'une fente en forme de L (306, 307).
